(19) Europäisches Patentamt / European Patent Office / Office européen des brevets

(11) **EP 4 233 922 B1**

(12) **EUROPEAN PATENT SPECIFICATION**

(45) Date of publication and mention
of the grant of the patent:
**12.11.2025 Bulletin 2025/46**

(21) Application number: **21886132.6**

(22) Date of filing: **25.10.2021**

(51) International Patent Classification (IPC):
**A61L 17/10** *(2006.01)*     **A61L 17/14** *(2006.01)*
**D01F 6/62** *(2006.01)*     **D01F 6/84** *(2006.01)*

(52) Cooperative Patent Classification (CPC):
(C-Sets available)
**A61L 17/105; A61L 17/14; D01F 6/625; D01F 6/84**
(Cont.)

(86) International application number:
**PCT/JP2021/039270**

(87) International publication number:
**WO 2022/092014 (05.05.2022 Gazette 2022/18)**

(54) **BIOABSORBABLE FIBROUS MEDICAL MATERIAL**

BIOABSORBIERBARES FASERIGES MEDIZINISCHES MATERIAL

MATÉRIAU MÉDICAL FIBREUX BIOABSORBABLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.10.2020 JP 2020178860**

(43) Date of publication of application:
**30.08.2023 Bulletin 2023/35**

(73) Proprietors:
- **MITSUBISHI GAS CHEMICAL COMPANY, INC.
Chiyoda-ku
Tokyo 100-8324 (JP)**
- **National University Corporation Tokai National
Higher Education and Research System
Nagoya-shi, Aichi 464-8601 (JP)**

(72) Inventors:
- **MAEHARA Akira
Niigata-shi, Niigata 950-3112 (JP)**
- **HIRATA Hitoshi
Nagoya-shi, Aichi 464-8601 (JP)**
- **MURAYAMA Atsuhiko
Nagoya-shi, Aichi 464-8601 (JP)**
- **NAKAGAWA Yasunobu
Nagoya-shi, Aichi 464-8601 (JP)**

(74) Representative: **Müller-Boré & Partner
Patentanwälte PartG mbB
Friedenheimer Brücke 21
80639 München (DE)**

(56) References cited:
CN-A- 102 586 936     JP-A- 2002 371 431
JP-A- 2003 328 230     JP-A- 2003 339 849
JP-A- 2007 046 050     JP-A- H0 489 065
JP-A- H06 336 523     US-A1- 2016 324 619

- TOSHIHISA TANAKA ET AL: "Formation of Highly
Ordered Structure in Poly[( R )-3-
hydroxybutyrate- c o -( R )-3-hydroxyvalerate]
High-Strength Fibers", MACROMOLECULES,
vol. 39, no. 8, 1 April 2006 (2006-04-01), US,
pages 2940 - 2946, XP055375587, ISSN:
0024-9297, DOI: 10.1021/ma0527505
- DAVID P. MARTIN AND SIMON F. WILLIAMS:
"Medical applications of poly-4-hydroxybutyrate:
a strong flexible absorbable biomaterial",
BIOCHEMICAL ENGINEERING JOURNAL,
ELSEVIER, AMSTERDAM, NL, vol. 16, 9
December 2002 (2002-12-09), NL
, pages 97 - 105, XP002740256, ISSN: 1369-703X,
DOI: 10.1016/S 1369-703X(03)00040-8

**(Cont. next page)**

(52) Cooperative Patent Classification (CPC): (Cont.)

C-Sets
**A61L 17/105, C08L 67/04**

**Description**

Technical Field

The present invention relates to a fibrous medical material using stretchable and bioabsorbable aliphatic polymer fibers, which allows easy knot formation and enables ligation that provides a small and hard-to-unravel knot.

Background Art

Sutures include monofilament sutures made of a single fiber and multifilament sutures made of a plurality of fibers. As a material for the sutures, a non-absorbable polymer or an absorbable polymer is used. Examples of the non-absorbable polymer includes polyethylene, polypropylene, nylon, silicone, Teflon, and silk. Examples of the absorbable polymer include homopolymers or copolymers produced by polymerizing glycolic acid, lactic acid, ε-caprolactone, dioxanone, or the like. On the other hand, it is known that a suture containing glycolic acid or lactic acid tends to cause a strong inflammatory reaction in an absorption process, and may be problematic in terms of biocompatibility.

Conventional bioabsorbable sutures include those used as multifilaments because they are made of stiff polymers such as polyglycolic acid (PGA) and poly(glycolide/L-lactide) copolymers, and those used as monofilaments with increased pliability as copolymers such as poly(glycolide/trimethylene carbonate) copolymers, poly(glycolide/ε-caprolactone) copolymers, poly-p-dioxanone, poly(glycolide/trimethylene carbonate/p-dioxanone) copolymers, poly(glycolide/trimethylene carbonate/ε-caprolactone) copolymers, poly(glycolide/L-lactide/trimethylene carbonate/ε-caprolactone) copolymers, and poly(L-lactide/ε-caprolactone) copolymers. These sutures are selectively used according to required strength, maintenance period for tensile strength, absorption period, site for application, tissue reaction, tissue damage, required elasticity, knot safety, operability, economy, infection resistance, physician experience, etc.

For example, Patent Document 1 describes a surgical suture composed of a monofilament yarn made of a copolymer of lactic acid and ε-caprolactone. Patent Document 2 describes a suture produced by melt spinning a glycolide/ε-caprolactone copolymer. Patent Document 3 describes a monofilament suture produced by composite spinning of a first polymer and a second polymer synthesized from one or more monomers selected from the group consisting of glycolide, glycolic acid, lactide, lactic acid, caprolactone, dioxanone, trimethylene carbonate, and ethylene glycol, wherein the first polymer and the second polymer each have a Young's modulus of 3.0 GPa or less. Patent Document 4 describes a synthetic composite biomaterial containing collagen, at least one organic polymer (polyglycolide, polylactide, copolymer of glycolide and lactide, polylactone, polyhydroxyalkanoic acid, etc.), and at least one active ingredient.

In addition, sutures made of a polyhydroxyalkanoate (sometimes referred to as PHA) have also been reported. For example, Patent Document 5 describes a polyester molded article containing a biodegradable polyester copolymer composed of a 3-hydroxybutyrate (sometimes referred to as 3HB) unit and a 4-hydroxybutyrate (sometimes referred to as 4HB) unit, wherein the 4-hydroxybutyrate unit has a content of greater than 60 mol% and 95 mol% or less. Patent Document 6 and Patent Document 7 describe a medical device including a suture made of a biocompatible polyhydroxyalkanoate. Patent Document 8 describes fibers containing a poly-4-hydroxybutyrate polymer, wherein the fibers have a tensile strength of greater than 126 MPa. Patent Document 9 and Patent Document 10 describe a polymer product produced by drawing a composition characterized by a biodegradable polyhydroxyalkanoate copolymer including at least two randomly repeating monomer units. Patent Document 11 describes a polyester copolymer composed of from 97 to 40 mol% of a 3-hydroxybutyrate unit and from 3 to 60 mol% of a 4-hydroxybutyrate unit and having a [η], as measured in chloroform at 30°C in a range of from 0.4 to 10.0 dL/g, and describes that the polyester copolymer is rich in flexibility and good in moldability, and that the produced molded articles such as fibers and films are pliable and tough. Patent Document 13 describes a degradable fiber with good resilience and a preparation method thereof, the fibers comprising a mixture of poly(3-hydroxybutyrate-co-4-hydroxybutyrate) and polyethylene vinyl acetate. However, none of the above related arts describes that a fibrous medical material having an elongation at break of a predetermined value or more and an elastic modulus (an initial elastic modulus in tension and an intermediate elastic modulus in tension which will be described later) of a predetermined value or less can provide a fiber that provides a small and hard-to-unravel knot.

Furthermore, a monofilament suture (MonoMax (trade name)) made of a homopolymer of 4-hydroxybutyric acid (also referred to as P(4HB)) has been developed (Patent Document 12 and Non-Patent Literatures 1 and 2). This MonoMax suture has been reported to have an elastic modulus of 485 MPa (Non-Patent Literature 2), which is considered to be a soft suture having an elastic modulus lower than that of PDSII made of poly-p-dioxanone (1370 MPa) and that of Monocryl made of a poly(glycolide/ε-caprolactone) copolymer (725 MPa). However, when actually used by a doctor, the suture is stiff and knots tend to loosen, and thus it is necessary to increase the number of knots.

Prior Art Documentst

Patent Documents

Patent Document 1: JP 2001-149462 A
Patent Document 2: JP 2011-6496 A
Patent Document 3: JP 4071661 B
Patent Document 4: JP 2019-505338 T
Patent Document 5: JP 06-336523 A
Patent Document 6: US 6867247 B
Patent Document 7: JP 5031144 B
Patent Document 8: JP 2007-525601 T
Patent Document 9: JP 2003-513130 T
Patent Document 10: JP 2003-513131 T
Patent Document 11: JP 1-48821 A
Patent Document 12: WO 2004/101002
Patent Document 13: CN 102 586 936 A

Non-Patent Literatures

Non-Patent Literature 1: BMC Surgery volume 8, Article number: 12 (2008)
Non-Patent Literature 2: International Journal of Polymer Science Volume 2012, Article ID 216137

Summary of Invention

Problem to be Solved by the Invention

In the case of using a suture in a surgical operation, it is a basic technique to form a knot for ligation. The characteristics of ease of knot formation and difficulty in unraveling of knots are required of sutures, and are similarly required not only of non-absorbable sutures but also of bioabsorbable sutures.

The multifilament suture is made by weaving fine fibers and does not have a smooth surface. Therefore, the suture has advantages of difficulty in unraveling of knots and rich flexibility. However, the suture, when passed through tissue, exhibits great invasion to the tissue, resulting in the tendency of poor knot lubrication, that is, the tendency of difficulty in knot lubrication because of a high friction coefficient. In addition, the multifilament suture unfortunately has a higher risk of infection than that of the monofilament suture due to the generation of fine capillaries. In this regard, the monofilament suture has a smooth surface, exhibits less invasion to tissue and is resistant to infection, but is disadvantageously incompliant, resulting in ease of loosening of knots. Therefore, for the monofilament suture, the ease of loosening of knots can be compensated by increasing the number of knots, but, as a result, the knots become large, leading to concern about the influence on the tissue. Also, when forming a knot, the physician often tends to tighten the knot with a strong force to form a firm and tight knot, because known monofilament sutures are stiff, impliable, and incompliant. Therefore, when the sutures are tightened with a strong force to form a knot, an excessive force is applied to a sutured part tissue, leading to concern about unintentional damage to the tissue. Therefore, there is a need for development of a suture which has high extensibility such that an appropriate tension is uniformly retained until tissue self-repairs while suppressing invasion to the tissue, and which is per se pliable and makes a knot difficult to unravel, and for development of a suture which allows easy formation of a knot which is difficult to unravel without being tightened with a strong force. Furthermore, there is a further need for development of a bioabsorbable suture which is bioabsorbable, allows easy formation of a knot, and provides a small and hard-to-unravel knot, so that suture removal and re-incision are unnecessary, and that reduction in foreign-body sensation given to the surrounding tissue by an embedded knot in subcutaneous suture, suture inside the body, or the like can be expected. Due to such a small and hard-to-unravel knot, it is expected that, when a surplus suture is cut off from the knot, the need to leave a cut end of the suture long is reduced, and that, in the case of a flexible suture, tingling irritation to tissue caused by a stiff cut end of the suture is also reduced.

In addition, the tissue may swell for various reasons. However, a known suture lacks stretchability, and thus cannot appropriately follow the swelling of the tissue, and an excessive tension is applied to tissue, which may cause a scar. There is also a need for development of a bioabsorbable suture having an elastic modulus closer to an elastic force of tissue, i.e., lower than an elastic modulus of a known suture, and having such stretchability as to shrink after stretching, so that, even when the tissue swells, the suture can stretch and disperse tension, and that, when the swelling is reduced, the suture shrinks and continuously contributes to wound adhesion.

A problem to be solved by the present invention is to provide a bioabsorbable fibrous medical material that enables formation of ligation which provides a small and hard-to-unravel knot even with a weak force. It is also a part of the problem to provide a stretchable bioabsorbable fibrous medical material that can follow movement of tissue.

Means for Solution to Problem

As a result of intensive studies to solve the above problems, the present inventors have found that it is possible to provide a bioabsorbable and stretchable fibrous medical material which allows easy knot formation, enables ligation providing a small and hard-to-unravel knot even with a weak force, and can decrease the number of knots itself, by using, as a raw material, a bioabsorbable aliphatic polymer, setting an elongation at break of a molded article provided by spinning and drawing the bioabsorbable aliphatic polymer to 75% or greater, setting an intermediate elastic modulus in tension to be lower than an initial elastic modulus in tension, setting the intermediate elastic modulus in tension to 400 MPa or less, and setting a residual strain rate after 100% deformation to 70% or less. The scope of the present invention is defined by the claims. The present invention is defined by independent claim 1, while the dependent claims concern the preferred embodiments. Any "embodiment" or "example" which is disclosed in the description but is not covered by the claims should be considered as presented for illustrative purpose only. Embodiments in the description relating to methods of treatment are not covered by the claims.

Advantageous Effects of Invention

The bioabsorbable fibrous medical material of the present invention has good operability, allows formation of a knot with a small force, and enables ligation which provides a small and hard-to-unravel knot.

Brief Description of Drawings

[FIG. 1] FIG. 1 is a schematic view for illustrating a surgical knot.
[FIG. 2] FIG. 2 is a schematic view for illustrating one single knot added onto a surgical knot.
[FIG. 3] FIG. 3 is an SEM image of a surface of a P(3HB-co-4HB) suture 2.5-0. P(3HB-co-4HB) means a copolymer of 3-hydroxybutyric acid and 4-hydroxybutyric acid.
[FIG. 4] FIG. 4 is an SEM image of a surgical knot of a P(3HB-co-4HB) suture 3-0.
[FIG. 5] FIG. 5 is a series of stereoscopic microscope images of a surgical knot of an opaque P(3HB-co-4HB) suture having 3-0 size.
[FIG. 6] FIG. 6 is a series of stereoscopic microscope images of a surgical knot of a colorless P(3HB-co-4HB) suture having 3-0 size.
[FIG. 7] FIG. 7 is a series of stereoscopic microscope images of a surgical knot of a colorless P(3HB-co-4HB) suture having 1 size.
[FIG. 8] FIG. 8 is a series of stereoscopic microscope images of a surgical knot of a white P(3HB-co-4HB) suture having 2.5-0 size.
[FIG. 9] FIG. 9 is an SEM image of a surface of a P(4HB) MonoMax (trade name) suture 2-0.
[FIG. 10] FIG. 10 is an SEM image of a surgical knot of the P(4HB) MonoMax (trade name) suture 2-0.
[FIG. 11] FIG. 11 is a series of stereoscopic microscope images of the surgical knot of the P(4HB) MonoMax (trade name) suture 2-0.
[FIG. 12] FIG. 12 is a series of stereoscopic microscope images of a surgical knot of a PDSII suture 3-0.
[FIG. 13] FIG. 13 is a series of stereoscopic microscope images of a surgical knot of a PDSII suture 4-0.
[FIG. 14] FIG. 14 is a graph showing tensile fracture strength/fracture elongation of a P(3HB-co-4HB) suture over an immersion time thereof in a buffer.
[FIG. 15] FIG. 15 is a graph showing a decrease in weight average molecular weight Mw of the P(3HB-co-4HB) suture over the immersion time thereof in the buffer.
[FIG. 16] FIG. 16 is a graph showing tensile fracture strength/fracture elongation of the P(3HB-co-4HB) suture over an implantation time thereof in a rat body.
[FIG. 17] FIG. 17 is a graph showing a decrease in weight average molecular weight Mw of the P(3HB-co-4HB) suture over the implantation time thereof in the rat body.
[FIG. 18] FIG. 18 is a series of photographs of states of a sutured part of a microminipig 7 weeks after suture with a bioabsorbable suture.
[FIG. 19] FIG. 19 is an image of an HE stained sample of a sutured part tissue with the P(3HB-co-4HB) suture.

[FIG. 20] FIG. 20 is an image of an HE stained sample of a sutured part tissue with a polyglyconate (PGA) suture.

[FIG. 21] FIG. 21 is an image of an HE stained sample of a sutured part tissue with a P(4HB) suture.

[FIG. 22] FIG. 22 is a graph showing an example of a stress-strain curve to break of a fiber of Example 1 in a tensile test (measured at a chuck-to-chuck distance of 1 cm).

[FIG. 23] FIG. 23 is a graph showing an example of a stress-strain curve to break of a fiber of Example 2 in a tensile test.

[FIG. 24] FIG. 24 is a graph showing an example of a stress-strain curve to break of a fiber of Example 3 in a tensile test.

[FIG. 25] FIG. 25 is a graph showing an example of a stress-strain curve to break of a fiber of Example 4 in a tensile test.

[FIG. 26] FIG. 26 is a graph showing stress-strain curves to 100% strain of the P(3HB-co-4HB) suture (3 cm) of Example 1 in a cycle test.

[FIG. 27] Figure 27 is a graph showing stress-strain curves to 100% strain of the P(3HB-co-4HB) suture (3 cm) of Example 2 in a cycle test.

[FIG. 28] FIG. 28 is a graph showing stress-strain curves to 100% strain of the P(3HB-co-4HB) suture (3 cm) of Example 3 in a cycle test.

[FIG. 29] FIG. 29 is a graph showing stress-strain curves to 100% strain of the P(3HB-co-4HB) suture (3 cm) of Example 4 in a cycle test.

[FIG. 30] Figure 30 is a graph showing stress-strain curves to 50% strain of the P(3HB-co-4HB) suture (12 cm) of Example 4 in a cycle test.

[FIG. 31] FIG. 31 is an image of an example of a cross-sectional view of the suture of Example 1.

[FIG. 32] FIG. 32 is an image of an example of the cross-sectional view of the suture of Example 1.

[FIG. 33] FIG. 33 is an image of an example of a cross-sectional view of the suture of Example 2.

[FIG. 34] FIG. 34 is an image of an example of a cross-sectional view of the suture of Example 3.

[FIG. 35] FIG. 35 is an SEM image of a surface of the P(3HB-co-4HB) suture of Example 4.

[FIG. 36] FIG. 36 is an SEM image of a cross-section of the P(3HB-co-4HB) suture of Example 4.

[FIG. 37] Figure 37 is an SEM image of a surgical knot of the P(3HB-co-4HB) suture of Example 4.

Embodiments of the Invention

The present invention will be described in detail below. Note that the following embodiments are examples for describing the present invention, and the present invention is not limited only to the embodiments.

The bioabsorbable fibrous medical material of the present invention is composed of a molded article provided by spinning and drawing a bioabsorbable aliphatic polymer. According to the claimed invention, the bioabsorbable aliphatic polymer is a polyhydroxyalkanoate composed of two or more types of hydroxyalkanoate units, wherein the hydroxyalkanoate units contain 3-hydroxybutyrate and 4-hydroxybutyrate, wherein the proportion of the 4-hydroxybutyrate unit relative to all monomer units is 10 mol% to 30 mol%.

<Bioabsorbable Aliphatic Polymer>

When a polymer is "bioabsorbable", the polymer is naturally decomposed by a hydrolysis reaction or an enzyme reaction after being placed inside or outside a living body, and the decomposition product is metabolized or excreted and thus disappears. In other words, the term "bioabsorbable" refers to having local disappearance and extracorporeal elimination properties.

The term "local disappearance property" means that a polymer is decomposed in a physiological environment within a predetermined number of days (e.g., 360 days, 240 days, 120 days, 60 days, or 30 days) and disappears from a local application area. As an example of the local disappearance property, when a sample corresponding to a polymer concentration of at least 1 mass% is put into physiological saline (pH 4 to 8) at 37°C, mixed using a rotor mixer, and visually observed, and the shape of the sample disappears within a predetermined number of days to form a transparent aqueous solution, the sample can be determined to have the local disappearance property. Alternatively, also when a sample is implanted in a body and is decomposed and disappears within a predetermined number of days, the sample can be determined to have the local disappearance property. The term "extracorporeal elimination property" means that, after disappearance of a material from an application site, the material can be eliminated out of the body without being excessively accumulated in organs such as kidney and liver. For example, when the material is decomposed to a molecular weight of 70,000 or less, and 40,000 or less in some cases, the sample can be determined to have the extracorporeal elimination property. Alternatively, after disappearance of the material from the application site, the material may become a partial decomposition product or a low molecular weight compound, be further metabolized even to water and carbon dioxide, and used in the body or eliminated out of the body.

<Polyhydroxyalkanoate>

The polyhydroxyalkanoate used in the present invention is biodegradable and bioabsorbable, and can have repeated stretchability (property of repeatedly stretching). The polyhydroxyalkanoate used in the present invention is a copolyester containing two or more types of monomer units. As the polymerization unit, a 4-hydroxybutyrate (sometimes referred to as 4HB) unit is contained in addition to the 3-hydroxybutyrate (sometimes referred to as 3HB) unit.

The weight average molecular weight of the polyhydroxyalkanoate determined by gel permeation chromatography, calibrated with polystyrene, is 300,000 or greater, 400,000 or greater, or 500,000 or greater after spinning. The weight average molecular weight determined by gel permeation chromatography, calibrated with polystyrene, may be 600,000 or greater, 700,000 or greater, 800,000 or greater, 900,000 or greater, 1,000,000 or greater, 1,100,000 or greater, 1,200,000 or greater, 1,300,000 or greater, 1,400,000 or greater, 1,500,000 or greater, 2,000,000 or greater, 3,000,000 or greater, or 4,000,000 or greater. The upper limit of the weight average molecular weight determined by gel permeation chromatography, calibrated with polystyrene, is not particularly limited and is typically 20,000,000 or less, 10,000,000 or less, 8,000,000 or less, 7,000,000 or less, 6,000,000 or less, 5,000,000 or less, 4,000,000 or less, or 3,000,000 or less. However, considering reduction in molecular weight due to thermal decomposition and excessively high viscosity at the time of melting, when melt-molding is performed, the weight average molecular weight determined by gel permeation chromatography, calibrated with polystyrene, is preferably 400,000 or greater and 2,500,000 or less, more preferably 500,000 or greater and 2,200,000 or less, and even more preferably 600,000 or greater and 2,000,000 or less.

The polyhydroxyalkanoate used in the present invention contains, as a polymerization unit, a 3-hydroxybutyrate (3HB) unit and a 4-hydroxybutyrate (4HB) unit. When the polyhydroxyalkanoate contains a 3HB unit and a 4HB unit, the polyhydroxyalkanoate may contain, as a polymerization unit, another polymerization unit other than the 3HB unit and the 4HB unit. Examples of such another polymerization unit include lactate (LA), glycolate (GA), 3-hydroxypropionate (3HP), 3-hydroxyvalerate (3HV), 4-hydroxyvalerate (4HV), 5-hydroxyvalerate (5HV), 4-hydroxyhexanoate (4HH), 5-hydroxyhexanoate (5HH), 6-hydroxyhexanoate (6HH), or 3-hydroxyhexanoate (3HH), or hydroxyalkanoates having 7 or more carbon atoms. Furthermore, not as a bipolymer but a terpolymer or multicomponent copolymer containing the polymerization unit(s) described above can be also used. Furthermore, the copolymer compositions described above can be mixed (blended) at a freely selected proportion and used.

In the present invention, the 3-hydroxybutyrate unit and the 4-hydroxybutyrate unit are represented by the following formulas.

3-Hydroxybutyrate unit: $-OCH(CH_3)CH_2C(=O)-$
4-Hydroxybutyrate unit: $-OCH_2CH_2CH_2C(=O)-$

The proportion of the 4-hydroxybutyrate unit relative to all monomer units is from 10 mol% to 30 mol%. The proportion of the 4-hydroxybutyrate unit relative to all monomer units is 10 mol% or greater, 11 mol% or greater, 12 mol% or greater, 13 mol% or greater, 14 mol% or greater, 15 mol% or greater, or 16 mol% or greater, and may be 17 mol% or greater, 18 mol% or greater, 19 mol% or greater, or 20 mol% or greater but 30 mol% or less, 29 mol% or less, 28 mol% or less, 27 mol% or less, 26 mol% or less, 25 mol% or less, 24 mol% or less, 23 mol% or less, 22 mol% or less, or 21 mol% or less.

The polyhydroxyalkanoate of the present invention may be any one selected from a random polymer, a block polymer, an alternating polymer, or a grafted polymer, but is preferably a random polymer.

[Method of Producing Polyhydroxyalkanoate]

In general, methods of synthesizing polyhydroxyalkanoates include fermentation synthesis methods (biosynthesis methods) and chemical synthesis methods. The method of producing the polyhydroxyalkanoate used in the present invention may be a fermentation synthesis method (biosynthesis method) or a chemical synthesis method, but is preferably a fermentation synthesis method (biosynthesis method) in order to provide a polyhydroxyalkanoate having a large molecular weight.

The chemical synthesis methods are methods of chemically synthesizing polyhydroxyalkanoates in accordance with typical organic synthesis techniques. As the chemical synthesis methods, specifically, for example, P(3HB-co-6HHx) can be synthesized by ring-opening polymerization of aliphatic lactones such as (R)-β-butyrolactone and ε-caprolactone in the presence of a catalyst (Abe et al., Macromolecules, 28, 7630 (1995)); P(3HB-co-4HB) and the like can be synthesized by ring-opening polymerization of aliphatic lactones such as (R)-β-butyrolactone and γ-butyrolactone in the presence of a catalyst (Hori et al., Polymer, 36, 4703 (1995)); and P(GA-co-LA) and the like can be synthesized by ring-opening polymerization of glycolide, lactide, and the like in the presence of a catalyst (Gilding et al., Polymer 20, 1459 (1979)). However, since a catalyst such as tin octylate is used, it is necessary to pay attention to an amount of the catalyst in the chemical synthesis of the bioabsorbable polyhydroxyalkanoate.

A fermentation synthesis method (biosynthesis method) is a method of biosynthesizing PHAs in accordance with a typical fermentation engineering technique. As the fermentation synthesis, specifically for example, a polyester containing 4HB of the present invention can be produced by culturing microorganisms having a P(3HB)-producing capability in the presence of ε-caprolactone (another name: 6-hexanolactone) or 6-hydroxyhexanoate, which is a saponified product thereof, or a salt thereof, γ-butyrolactone or 4-hydroxybutyrate, which is a saponified product thereof, or a salt thereof, a butyric acid derivative such as 4-chlorobutyrate or 4-bromobutyrate, or an even-chain α,ω-alkanediol having 4 or more carbons such as 1,4-butanediol, 1,6-hexanediol, 1,8-octanediol, 1,10-decanediol, or 1,12-dodecanediol as a carbons source (Saito et al., Polymer International 39, 169 (1996), and WO 2019/044837). P(3HB-co-4HB) copolymers having various 4HB ratios can be produced by appropriately changing the type and feed proportion of the carbon source to be used. The PHAs thus furnished by the enzymatic reaction of the organism do not contain a metal catalyst such as tin octylate as in the chemical synthesis method, and are advantageous in this sense. In the fermentation synthesis method, a genetically recombinant bacterium may be used, or a genetically non-recombinant bacterium may be used.

As the method for extraction of a polyhydroxyalkanoate from a bacterial cell, a solvent extraction method of extracting PHA with a halogenated hydrocarbon solvent such as chloroform and precipitating it with a poor solvent such as hexane or methanol may be used as known, or a water-based extraction method may be used as described in JP 04-061638 A, JP 07-177894 A, and WO 2004029266. In the case of producing a polyhydroxyalkanoate to be used in a living body, a step capable of removing impurities such as proteins derived from bacterial cells and endotoxin may be employed in a polyhydroxyalkanoate purification step, and the purified polyhydroxyalkanoate may be depyrogenated using a peroxide as described in US 6245537.

<Measurement of Molecular Weight of Polyhydroxyalkanoate (Gel Permeation Chromatography (GPC) Method)>

The measurement of the molecular weight of a polyhydroxyalkanoate can be performed by gel permeation chromatography method as will be described below.

The polyhydroxyalkanoate was adjusted to approximately 0.5 mg/mL by adding chloroform and dissolved at 60°C for from 2 to 4 hours, and cooled to room temperature. Insoluble substances were filtered and removed by using a PTFE filter having a pore diameter of 0.2 μm to produce a measurement sample. Conditions for GPC are as shown below.

Instrument: HPLC Prominence system, available from Shimadzu Corporation
Column: Shodex K-806L (two columns in series), available from Showa Denko K.K.
Column temperature: 40°C
Mobile phase: Chloroform (1 mL/min)
Detector: RI (40°C)
Standards: Shodex polystyrene molecular weight standards (6,870,000 to 1,270)
Injection amount: 60 μL
Analysis time: 30 minutes

<Production of Molded Article from Bioabsorbable Aliphatic Polymer Such As Polyhydroxyalkanoate>

The method for producing a molded article (such as a stretchable suture) from a bioabsorbable aliphatic polymer such as polyhydroxyalkanoate is not particularly limited as long as the solidification or crystallization of the bioabsorbable polyhydroxyalkanoate, having a low crystallization speed has proceeded to such an extent that the polymer can be drawn from an amorphous state after melting, and a drawing operation is applied in a state in which fine microcrystals are present to impart stretchability. For example, a molded article (stretchable suture or the like) can be produced according to the methods described in Japanese Patent Applications Nos. 2019-90739, 2020-096144 and 2020-096145.

Specifically, the stretchable suture can be produced by melt-molding the bioabsorbable polyhydroxyalkanoate and then drawing the polymer in a state in which microcrystals are formed at room temperature or at a temperature at which crystallization proceeds for an appropriate waiting time in order to promote crystallization, or by melt-molding the polymer in a partially molten state and drawing the polymer in a state in which microcrystals remain. When the bioabsorbable polyhydroxyalkanoate is melt-molded, additives may be added as long as the effects of the present invention are not impaired.

Examples of the additives include one or more selected from antioxidants, thermal stabilizers (e.g., hindered phenols, hydroquinone, phosphites and substituents thereof), ultraviolet absorbers (e.g., resorcinol, and salicylate), anti-colorants (e.g., phosphite and hypophosphite), lubricants, release agents (e.g., montanic acid and metal salts thereof, esters thereof, half esters thereof, stearyl alcohol, stearamide and polyethylene waxes), colorants (e.g., dyes or pigments), carbon black as a conductive or colorant, plasticizers, flame retardants (e.g., bromine-based flame retardant, phosphorus-based flame retardant, red phosphorus, and silicone-based flame retardant), flame retardant aids, and antistatic agents.

A method of formulating an additive into the bioabsorbable polyhydroxyalkanoate is not particularly limited, and includes dry blend, solution blending, and addition during chemical polymerization of the bioabsorbable polyhydroxyalkanoate.

The bioabsorbable polyhydroxyalkanoate is subjected to melt-molding. According to the claimed invention, the melt-molding is spinning (melt extrusion spinning or partial melt extrusion spinning), and is accompanied by a drawing operation for imparting stretchability.

In the melt extrusion spinning, a small plunger type melt extrusion apparatus at a laboratory level can also be used, but a large apparatus such as a single screw type spinning apparatus or a twin screw type spinning apparatus, which is industrially used, can also be used.

The number of times of melt-molding is not particularly limited, but melt-molding can be performed only once. Prior to melt-molding, heating treatment mediated by gas, liquid or solid may or may not be carried out in order to arbitrarily adjust the main lamellar thickness of the raw material bioabsorbable aliphatic polymer such as polyhydroxyalkanoate (Japanese Patent Application No. 2020-96145).

In the present invention, a solidification step after molding can be performed in a molding die, in the air, or in a liquid (e.g., in water). That is, solidification can be performed by cooling the molten bioabsorbable polyhydroxyalkanoate in a molding die, in the air, or in water. Preferably, the molten bioabsorbable polyhydroxyalkanoate can be cooled in a molding die or in the air. When cooling is performed in the air, the temperature and humidity of the air can also be controlled, but cooling may be performed at room temperature without special temperature control. In addition, a gas having a changed component composition (nitrogen, oxygen, carbon dioxide, moisture concentration, or the like) in the air may be used, or cooling may be performed in an environment where a rare gas (helium, neon, argon, or the like) or the like may be added or circulated. When cooling is performed in a liquid, the temperature and components (water, alcohols, glycerol, and the like) of the liquid may be arbitrarily changed.

Since the bioabsorbable polyhydroxyalkanoate in a molten amorphous state is in a solution state at a temperature equal to or higher than the glass transition point and has a low crystallization speed, and thus even if the polymer is drawn as it is, the molecules flow and are immediately cut. However, if the polymer is partially crystallized and drawn in a solidified state, the polymer chain can be oriented and drawn.

Examples of the molded article of the bioabsorbable polyhydroxyalkanoate produced by the method of the present invention include various fibers such as a drawn yarns and super drawn yarns, and a suture can be indicated as an example thereof. The suture may be either a monofilament suture made of a single fiber or a multifilament suture made of a plurality of fibers. In the case of producing a suture, a diameter of the suture is not particularly limited, and is typically 1 mm or less, and may be 0.8 mm or less, 0.6 mm or less, 0.5 mm or less, 0.4 mm or less, or 0.3 mm or less, and a lower limit of the yarn diameter is typically 0.001 mm or greater. The produced fibers may be used to be woven, knitted, and processed into a network, or to be woven into a three dimensional structure. Furthermore, it is also possible to produce composite fibers or three dimensional composites by combining the fibers with other fibers or materials.

In order for the produced fibers to exhibit stretchability, the fibers extruded by melt spinning or partial melt spinning are subjected to drawing treatment so that $\alpha$-crystals forming lamellar crystals in the fibers are oriented along the fiber direction and that lamellae composed of the $\alpha$-crystals are layered in a direction perpendicular to the fiber axis. Amorphous layers and tie molecules between the lamellae can be deformed by tension, and $\beta$-crystals having a plane zigzag stretched chain structure can be increased, and reduced or eliminated by removal of load to exhibit an elastic response (Japanese Patent Application No. 2019-90739).

The produced fibers exhibit stretchability without being subjected to a heat treatment (annealing treatment) at a temperature equal to or higher than the glass transition point and at which the fibers do not melt, but may be subjected to the heat treatment.

<Elongation at Break>

The elongation at break of the fibrous medical material of the present invention is 180% or greater, and particularly preferably 200% or greater. An upper limit of the elongation at break is not particularly limited and is typically 1000% or less.

The elongation at break can be measured by conducting a tensile test to break of the fiber by using the fibrous medical material having a length of 3 cm and a fiber diameter of from approximately 0.1 to 0.4 mm and a tensile tester AGS-50NX (available from Shimadzu Corporation) under conditions of a temperature of 23°C, a test speed of 10 mm/min, and an initial length (chuck-to-chuck distance) of 10 mm. When a sample having a sufficient length can be used, it is preferable to secure the chuck-to-chuck distance of 10 cm for the test.

<Porosity>

The fibrous medical material of the present invention may or may not have gaps. Preferably, the fibrous medical material of the present invention has gaps. When the fibrous medical material of the present invention has gaps, the range of the porosity is not particularly limited, and is preferably from 5 to 55%, more preferably from 10 to 50%, and even more preferably from 20 to 45%.

A method for measuring the porosity is not particularly limited, and, for example, the porosity can be measured by observing a cross section of the fibrous medical material with a scanning electron microscope and performing image analysis of the cross-sectional view. Software such as, but not limited to, ImageJ (an image processing program developed at the National Institutes of Health, USA) may be used for the image analysis of the cross-sectional view.

<Initial Elastic Modulus in Tension>

When the elastic modulus in tension at a strain ranging from 0.05% to 0.25% is defined as an initial elastic modulus in tension, the initial elastic modulus in tension of the fibrous medical material of the present invention is preferably 1000 MPa or less, more preferably 600 MPa or less, still more preferably 480 MPa or less, even more preferably 400 MPa or less, still even more preferably 300 MPa or less, and particularly preferably 200 MPa or less. A lower limit of the initial elastic modulus in tension is not particularly limited, and is typically 5 MPa or greater, and may be 10 MPa or greater.

<Intermediate Elastic Modulus in Tension>

When the elastic modulus in tension at a strain ranging from 0.25% to 10% is defined as an intermediate elastic modulus in tension, in the present invention, the intermediate elastic modulus in tension is lower than the initial elastic modulus in tension. The intermediate elastic modulus in tension of the fibrous medical material of the present invention is 400 MPa or less, more preferably 300 MPa or less, even more preferably 250 MPa or less, still even more preferably 200 MPa or less, and particularly preferably 150 MPa or less. A lower limit of the intermediate elastic modulus in tension is not particularly limited, and is typically 5 MPa or greater, and may be 10 MPa or greater.

The elastic modulus can be measured using, for example, a tensile tester. The chuck-to-chuck distance of the tensile tester is set to from 1 to 10 cm, and a test piece is fixed to a fixing tool using 1 cm above and below. A tensile speed is set to 10 mm/min. The initial elastic modulus in tension can be calculated from a slope of the stress-strain curve at a strain ranging from 0.05% to 0.25%, and the intermediate elastic modulus in tension can be calculated from a slope of the stress-strain curve at a strain ranging from 0.25% to 10%, for example. The strain section used for the intermediate elastic modulus in tension is larger than a strain section used for the calculation of the initial elastic modulus in tension, and a portion thereof close to the strain used for the calculation of the initial elastic modulus in tension may be arbitrarily changed. In the present specification, the elastic modulus in tension at a strain ranging from 0.25% to 10% is defined as the intermediate elastic modulus in tension.

<Residual Strain Rate After 100% Deformation>

A residual strain rate of the fibrous medical material of the present invention after 100% deformation is 70% or less, preferably 60% or less, and more preferably 50% or less. A lower limit of the residual strain rate after 100% deformation is not particularly limited, and is typically 5% or greater, and may be 10% or greater, 20% or greater, or 30% or greater.

<Residual Strain Rate After 50% Deformation>

A residual strain rate of the fibrous medical material of the present invention after 50% deformation is preferably 40% or less, more preferably 30% or less, and even more preferably 20% or less. A lower limit of the residual strain rate after 50% deformation is not particularly limited, and is typically 5% or greater, and may be 10% or greater, 20% or greater, or 30% or greater.

In the case where the following operations are repeated: conducting a cycle test of a fiber having a length 3 cm using a tensile tester under conditions of a temperature of 23°C and an initial length of 10 mm, stretching the fiber at a tensile speed of 20 mm/min to a strain of 100% (20 mm which is a length 2 fold the initial length, i.e., a displacement length of 10 mm), subsequently moving a gripper to the original length at the same speed, and shrinking the fiber,

a tensile elongation recovery rate $R_{100}$(%) is expressed as follows:

$$R_{100} = [20 - (X_{100} + 10)]/10 \times 100,$$

when a displacement length at the beginning of second elongation (i.e., approximately equal to the end of first shrinkage) is $X_{100}$ mm.

A residual strain rate $S_{100}$ (%) is expressed as follows:

$$S_{100} = 100 - R_{100}.$$

Similarly, in the case where the following operations are repeated: conducting a cycle test of a fiber having a length 12 cm using a tensile tester under conditions of a temperature of 23°C and an initial length of 100 mm, stretching the fiber at a tensile speed of 100 mm/min to 50% strain (150 mm which is a length 1.5 fold the initial length, i.e., a displacement length of 50 mm), subsequently moving a gripper to the original length at the same speed, and shrinking the fiber,

a tensile elongation recovery rate $R_{50}$ (%) is expressed as follows:

$$R_{50} = [150 - (X_{50} + 100)]/50 \times 100,$$

when a displacement length at the beginning of second elongation (i.e., approximately equal to the end of first shrinkage) is $X_{50}$ mm.

A residual strain rate $S_{50}$ (%) is expressed as follows:

$$S_{50} = 100 - R_{50}.$$

A tensile elongation recovery rate $R_a$ (%) is expressed by the following general formula:

$$R_a = [Y + Y \times a/100 - (X_a + Y)]/(Y \times a/100) \times 100,$$

when the chuck-to-chuck distance is Y, strain given at an initial stage is a%, and the displacement length at the beginning of the second elongation (i.e., approximately equal to the end of the first shrinkage) is $X_a$ mm.
A residual strain rate $S_a$ (%) is expressed as follows:

$$S_a = 100 - R_a.$$

<Diameter of Largest Fine Pore>

For the fibrous medical material of the present invention, a diameter of a largest fine pore (hole, fine pore, pore, void, gap, or hollow), as measured by microscopic observation of a cross section orthogonal to a fiber axis direction, is preferably 100 $\mu$m or less, more preferably 75 $\mu$m or less, and even more preferably 50 $\mu$m or less. A lower limit of the diameter of the largest fine pore (hole, fine pore, pore, void, gap, or hollow) is not particularly limited, and is typically 0.1 $\mu$m or greater, and may be 0.2 $\mu$m or greater, or 1 $\mu$m or greater. In addition, the number of holes per cross section is not limited, and may be one or more, and the respective holes may be independent or connected.
The diameter of the largest fine pore of the fibrous medical material can be measured, for example, by the following method.
An image in which holes inside the fiber can be identified was captured using a scanning electron microscope or the like; the image was analyzed using ImageJ (an image processing program developed by the National Institutes of Health, USA); and diameters of the fine pores was calculated.

<Ratio of Major Axis Length to Minor Axis Length>

The cross-sectional shape of the fibrous medical material of the present invention is not necessarily circular, and examples thereof include an ellipse, a polygon, a free curve, and a combination thereof. In the case of a circular shape, the yarn diameter can be measured by measuring the yarn width. For example, in the case of an elliptical shape, a ratio of a major axis length to a minor axis length (major axis length/minor axis length) may be 1.0 or greater, 1.1 or greater, or 1.2 or greater. An upper limit of the ratio of the major axis length to the minor axis length (major axis length/minor axis length) is not particularly limited, and is typically 3.0 or less, and may be 2.0 or less.

A ratio of the major axis length to the minor axis length (major axis length/minor axis length) in a cross section in a width direction of the fibrous medical material can be measured by the following method.

The minor axis length and the major axis length were measured using a dial thickness gauge (TECLOCK CO., LTD., Model SM-1201L, scale: 0.001 mm). Measurement was performed at three positions (1/4, 1/2, and 3/4 of the total length) of the yarn to be measured in a fiber direction in which the thickness is smallest, and an average value among them was defined as minor axis length. The major axis length was measured by lightly deforming the fiber into a U-shape so that the fiber does not crease, setting the major axis to be perpendicular between gauges, measuring the major axis lengths at three positions (1/4, 1/2, and 3/4 of the total length) of the yarn to be measured, and taking an average value among them as major axis length.

The present invention will be described more specifically hereinafter using examples, but the present invention is not limited to the examples.

Examples

In the following Examples and Comparative Examples, a knot of a suture and a side surface or cross section of the suture were observed with an electron microscope. Prior to observation, a suture cut into an appropriate size with a razor was placed on a sample table and coated with an osmic thin film using an osmic plasma coater, NL-OPC80NS (Nippon Laser Denshi K.K.). Observation was performed using a field emission type scanning electron microscope JSM-7610F (JEOL Ltd.) under the condition of an acceleration voltage of 5.0 kV. In addition, with respect to cross sections of some of sutures, sutures cut into an appropriate size with a razor were each placed on a sample table, and vapor-deposited with platinum (Pt) using an ion sputter, E1045 (Hitachi High-Tech Corporation). Thereafter, observation was performed using a thermal electron gun type low vacuum scanning electron microscope TM4000plus (Hitachi High-Tech Corporation) under the condition of an acceleration voltage of 5.0 kV.

<Example 1>

P(3HB-co-14.8 mol% 4HB) having a weight average molecular weight Mw of 970,000 was melt extruded using a plunger-type melt extrusion spinning apparatus IMC-19F8 (Imoto Machinery Co., Ltd.) with approximately 5 g of PHA charged into a cylinder and a die having a die diameter of 1 mm by heating at 170°C for 5 minutes (extrusion rate: 1 mm/sec), and the resulting yarns were wound up on a bobbin having a diameter of 114 mm at a speed of 5 rpm so that the yarns did not overlap with each other. After winding, the yarn was allowed to solidify at room temperature (23°C) for 60 minutes, partially crystallized, and then manually subjected to hot pin drawing at 60°C to prepare a slightly flattened and whitened stretchable monofilament yarn at a draw ratio of approximately from 5 to 10 times. The diameter of the prepared stretchable monofilament yarn was measured using a dial thickness gauge (TECLOCK CO., LTD., SM-1201L type, mesh size: 0.001 mm) or a dial gauge (OZAKI MFG.CO.,LTD., 5B-HG type, mesh size: 0.001 mm). Diameters at three positions (1/4, 1/2, and 3/4) of the yarn cut into 10 cm were measured, and an average value among them was used as yarn diameter. When the cross section of the P(3HB-co-4HB) yarn was not necessarily circular but elliptical or flat, the minor axis length and the major axis length were measured, and the cross sectional area was calculated as an ellipse and used for the evaluation of a tensile test. The cross section of the yarn of Example 1 in the present specification has a collapsed shape, not a circular shape, due to winding of the molten polymer in an amorphous state and hot pin drawing, and an average length of the minor axis length and the major axis length is indicated as the yarn diameter in Table 1. The hot pin drawing is a method in which a fiber is drawn while being pressed against a heated metal pin.

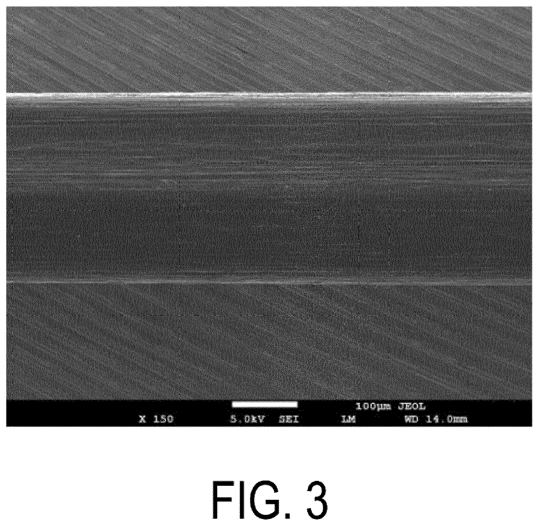

The surface of the yarn was observed was observed using a scanning electron microscope (FIG. 3).

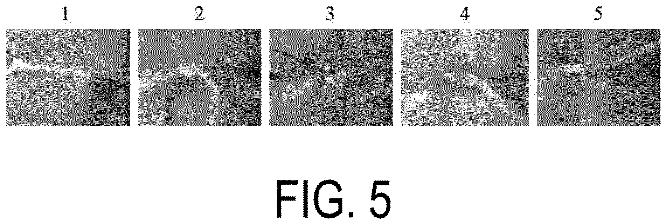

Using this yarn, a knot was formed by surgical knotting in an artificial skin sheet made of a soft elastomer, and fastened by using a force gauge (Standard Model Digital Force Gauge: ZTS-100N, IMADA CO., LTD) with a force of approximately 5 N. The knot was photographed with a camera ((DP26, OLYMPUS. CO., LTD) attached to a stereoscopic microscope (SZX7, OLYMPUS. CO., LTD), and the size of the knot (perimeter of the knot and area surrounded by the perimeter) was analyzed using image analysis software (cellSens, OLYMPUS. CO., LTD) (FIG. 5). The knot was three dimensional, but it was difficult to accurately estimate the volume. Therefore, [area surrounded by perimeter] and [area surrounded by perimeter/yarn diameter] were used as indexes of the knot size. Although the [area surrounded by perimeter] may vary depending on the direction in which the knot is viewed, five knots were randomly analyzed, and an average value therefor was treated.

The results are shown in Table 1 (Example 1).

The average value of the short-direction yarn diameter of the slightly flat suture made of P(3HB-co-14.8 mol% 4HB) of Example 1 was 0.205 mm, the long-direction yarn diameter thereof was 0.352 mm, and the average yarn diameter between in the short direction and in the long direction was 0.281 mm. The knot size of the surgical knot fastened with a force of 5 N was 3.46 mm for the average perimeter and 0.688 $mm^2$ for the average area of the region surrounded by the perimeter. The average value of the [area surrounded by perimeter/yarn diameter] as the index of the knot size was 2.45. The weight average molecular weight Mw of the PHA after spinning was 320,000.

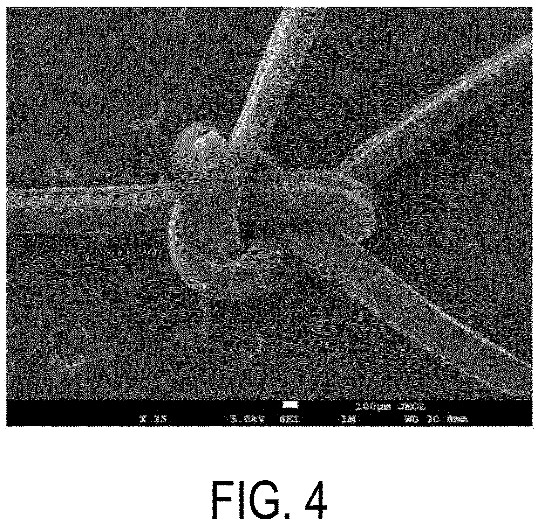

FIG. 4 shows the result of observation of the state of a surgical knot with a scanning electron microscope. FIG. 5 shows a series of optical microscope images of a surgical knot used for calculating a numerical value associated with the knot size.

[Table 1]

| | Material | Test No. | Yarn diameter*1 (mm) | Perimeter*2 (mm) | Area surrounded by perimeter*3 (mm$^2$) | Area surrounded by perimeter/yarn diameter |
|---|---|---|---|---|---|---|
| Example 1 (with void) | P(3HB-co-14.8 mol% 4HB) Mw 320,000 | A1-1 | 0.276 | 2.76 | 0.508 | 1.84 |
| | | A1-2 | 0.261 | 3.16 | 0.616 | 2.36 |
| | | A1-3 | 0.312 | 3.91 | 0.853 | 2.73 |
| | | A1-4 | 0.282 | 4.05 | 0.917 | 3.26 |
| | | A1-5 | 0.274 | 3.45 | 0.546 | 1.99 |
| | | Average | 0.281 | 3.46 | 0.688 | 2.45 |

*1 Average diameter between short-direction yarn diameter (minor axis length) and long-direction yarn diameter (major axis length) of cross-sectional ellipse
*2 Perimeter of outline of knot when viewed from one direction
*3 Area of region surrounded by perimeter of outline of knot when viewed from one direction

<Example 2>

P(3HB-co-15.3 mol% 4HB) having a weight average molecular weight Mw of 700,000 was used, and a plunger (piston) type melt viscosity measuring apparatus flow tester CFT-500D (Shimadzu Corporation) was used as a melt spinning apparatus. The piston diameter was 11.282 mm (piston cross-sectional area: 1 cm$^2$). Approximately 1 g of the PHA was charged into a cylinder. Partial melt spinning was performed at 150°C, using a die (nozzle) having a hole diameter of 1 mm and a hole length of 1 mm, after a remaining heat time of 120 seconds. A weight used was 2.5 kg, and a load of 3 kg in total was applied by the weight and a fishing tackle, and the polymer was extruded with an extrusion force of 2.942 MPa applied to the piston cross-sectional area of 1 cm$^2$ by an increase in force by pulleys. The PHA shows a melting peak from 85°C even to around 155°C in differential scanning calorimeter analysis (DSC) and is not completely molten but partially molten at a melting temperature of 150°C. The crystals remaining undissolved in the partial melt spinning and the extruded polymer molten and fluidized were already semi-solidified immediately after the extrusion, and the extruded polymer was manually drawn approximately from 5 times to 10 times to prepare a substantially transparent stretchable monofilament yarn, and the diameter of the yarn was measured using a dial thickness gauge (TECLOCK CO., LTD., SM-1201L type, mesh size: 0.001 mm) or a dial gauge (OZAKI MFG.CO.,LTD., 5B-HG type, mesh size: 0.001 mm). Diameters at three positions (1/4, 1/2, and 3/4) of the yarn cut into 10 cm were measured, and an average value among them was used as yarn diameter. The cross section of the yarn was approximately circular.

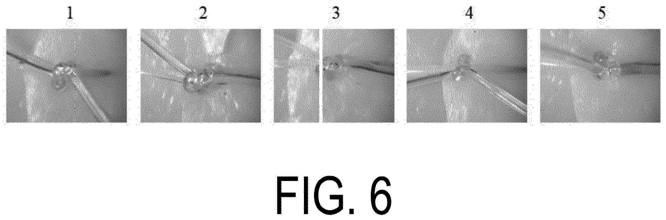

Using the yarn having size 3-0, a knot was formed by surgical knotting in the same manner as in Example 1, and the size of the knot was analyzed with a stereoscopic microscope (FIG. 6). The results are shown in Table 2 (Example 2).

The average value of the diameter of the suture made of P(3HB-co-15.3 mol% 4HB) of Example 2 was 0.207 mm (approximately circular, major axis length/minor axis length < 1.2), which was a yarn diameter corresponding to 3-0 according to the USP (United States Pharmacopia) standard. The knot size of the surgical knot fastened with a force of approximately 5 N was 3.61 mm for the average perimeter and 0.744 mm$^2$ for the average area of the region surrounded by the perimeter. The average value of the [area surrounded by perimeter/yarn diameter] as the index of the knot size was 3.59, which was slightly larger than that in Example 1. The weight average molecular weight Mw of the PHA after spinning was 470,000.

[Table 2]

| | Material, USP size | Test No. | Yarn diameter (mm) | Perimeter*1 (mm) | Area surrounded by perimeter*2 (mm $^2$) | Area surrounded by perimeter/yarn diameter |
|---|---|---|---|---|---|---|
| Example 2 (without void) | P(3HB-co-15.3% 4HB), Mw 470,000 3-0 size | A2-1 | 0.205 | 3.52 | 0.613 | 2.99 |
| | | A2-2 | 0.199 | 4.29 | 1.008 | 5.06 |
| | | A2-3 | 0.215 | 3.54 | 0.767 | 3.57 |
| | | A2-4 | 0.215 | 2.94 | 0.469 | 2.18 |
| | | A2-5 | 0.202 | 3.76 | 0.863 | 4.27 |
| | | Average | 0.207 | 3.61 | 0.744 | 3.59 |
| *1 Perimeter of outline of knot when viewed from one direction *2 Area of region surrounded by perimeter of outline of knot when viewed from one direction | | | | | | |

<Example 3>

P(3HB-co-15.3 mol% 4HB) having a weight average molecular weight Mw of 750,000 was used, and a plunger (piston) type melt viscosity measuring apparatus flow tester CFT-500D (Shimadzu Corporation) was used as a melt spinning apparatus. The piston diameter was 11.282 mm (piston cross-sectional area: 1 cm$^2$). Approximately 1 g of the PHA was charged into a cylinder. Melt spinning was performed at 170°C, using a die (nozzle) having a hole diameter of 1 mm and a hole length of 1 mm, after a remaining heat time of 120 seconds. A weight used was 2.5 kg, and a load of 3 kg in total was applied by the weight and a fishing tackle, and the polymer was extruded with an extrusion force of 2.942 MPa in the same manner as in Example 2. The PHA shows a melting peak from 60°C even to around 170°C in differential scanning calorimeter analysis (DSC), and is considered to be almost completely molten at a melting temperature of 170°C. The extruded fiber was suspended in a straight state without being wound around a bobbin, allowed to solidify at room temperature (23°C) for 30 minutes, partially crystallized, and then manually drawn at a draw ratio of approximately 5 times to prepare a transparent stretchable monofilament yarn, and the yarn diameter thereof was measured in the same manner as in Example 2.

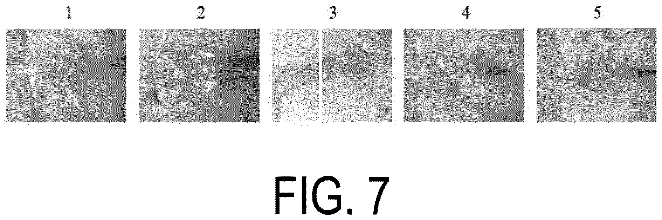

Using the yarn having 1 size, a knot was formed by surgical knotting in the same manner as in Example 1, and the size of the knot was analyzed with a stereoscopic microscope (FIG. 7). The results are shown in Table 3 (Example 3).

The average value of the diameter of the suture made of P(3HB-co-15.3 mol% 4HB) of Example 3 was 0.406 mm (approximately circular, major axis length/minor axis length < 1.2), which was a yarn diameter corresponding to 1 according to the USP standard. The knot size of the surgical knot fastened with a force of approximately 5 N was 4.87 mm for the average perimeter and 1.35 mm$^2$ for the average area of the region surrounded by the perimeter. The average value of the [area surrounded by perimeter/yarn diameter] as the index of the knot size was 3.33, which was equivalent to that in Example 2. The weight average molecular weight Mw of the PHA after spinning was 450,000.

As shown in FIGS. 5, 6 and 7, the knots of the stretchable yarns provided from P(3HB-co-4HB) indicated in Examples 1, 2 and 3 were tightly tied with no capillaries between the yarns, and Example 1 was slightly smaller, but Examples 2 and 3 were almost equivalent in terms of the [area surrounded by perimeter/yarn diameter] as the index of the knot size.

[Table 3]

|  | Material, USP size | Test No. | Yarn diameter (mm) | Perimeter*1 (mm) | Area surrounded by perimeter*2 (mm2) | Area surrounded by perimeter/yarn diameter |
|---|---|---|---|---|---|---|
| Example 3 (without void) | P(3HB-co-15.3% 4HB) Mw 450,000 1 size | A3-1 | 0.392 | 4.57 | 1.15 | 2.92 |
|  |  | A3-2 | 0.415 | 5.67 | 1.70 | 4.09 |
|  |  | A3-3 | 0.412 | 4.22 | 1.01 | 2.46 |
|  |  | A3-4 | 0.413 | 5.62 | 1.78 | 4.30 |
|  |  | A3-5 | 0.400 | 4.24 | 1.13 | 2.83 |
|  |  | Average | 0.406 | 4.87 | 1.35 | 3.33 |
| *1 Perimeter of outline of knot when viewed from one direction *2 Area of region surrounded by perimeter of outline of knot when viewed from one direction ||||||||

<Example 4>

The yarns of Examples 1 to 3 were prepared by manual drawing using a small plunger type melt extrusion apparatus at a laboratory level. P(3HB-co-16.0 mol% 4HB) copolymers having a weight average molecular weight Mw of 560,000 were partially melt spun using an industrially used single-screw spinning/drawing device having a diameter of 16 mm and a die having a diameter of 1 mm, with the temperature range of the extruder set to from 145 to 160°C, and extruded at a speed of 0.9 g/min. The extruded fiber made of a mixture of the undissolved crystals and the fluidized polymer was once passed through water at 50°C, and then wound and drawn (draw ratio: approximately 9 times) with multistage rollers at room temperature of 23°C in the air to provide a stretchable yarn. Using the provided yarn having a circular cross section, the yarn diameter was measured in the same manner as in Example 2.

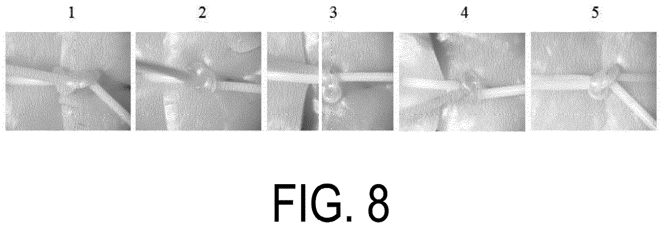

Using the yarn of the 2.5-0 standard, a knot was formed by surgical knotting in the same manner as in Example 1, and the size of the knot was analyzed with a stereoscopic microscope (FIG. 8). The results are shown in Table 4 (Example 4).

The average value of the diameter of the suture used in the evaluation of the size of the knot of the suture made of P(3HB-co-16.0 mol% 4HB) of Example 4 was 0.256 mm (approximately circular, major axis length/minor axis length < 1.2), which was a yarn diameter corresponding to 2.5-0 according to the USP standard. The knot size of the surgical knot fastened with a force of approximately 5 N was 4.03 mm for an average circumference and 0.843 mm$^2$ for an average area of a region surrounded by the circumference. The average value of the [area surrounded by circumference/yarn diameter] as the index of the knot size was 3.29, which was equivalent to those in Examples 2 and 3. The weight average molecular weight Mw of the PHA after spinning was 350,000.

As shown in FIGS. 5, 6, 7 and 8, the knots of the stretchable yarns provided from P(3HB-co-4HB) indicated in Examples 1, 2, 3 and 4 were tightly tied with no capillaries between the yarns, and the [area surrounded by circumference/yarn diameter] as the index of the knot size of Example 1 was slightly smaller than those of the other Examples, but those of Examples 2, 3 and 4 were almost equivalent.

[Table 4]

|  | Material, USP size | Test No. | Yarn diameter (mm) | Perimeter*1 (mm) | Area surrounded by perimeter*2 (mm2) | Area surrounded by perimeter/yarn diameter |
|---|---|---|---|---|---|---|
| Example 4 (without void) | P(3HB-co-16.0% 4HB), Mw 350,000 2.5-0 size | A4-1 | 0.268 | 4.99 | 1.06 | 3.96 |
|  |  | A4-2 | 0.261 | 4.06 | 1.00 | 3.82 |
|  |  | A4-3 | 0.250 | 3.77 | 0.736 | 2.90 |
|  |  | A4-4 | 0.244 | 3.52 | 0.694 | 2.84 |
|  |  | A4-5 | 0.258 | 3.79 | 0.747 | 2.86 |
|  |  | Average | 0.256 | 4.03 | 0.843 | 3.29 |
| *1 Perimeter of outline of knot when viewed from one direction *2 Area of region surrounded by perimeter of outline of knot when viewed from one direction ||||||||

<Comparative Example 1>

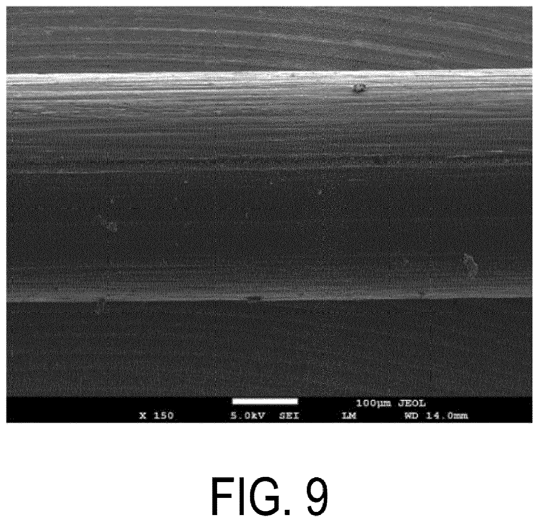

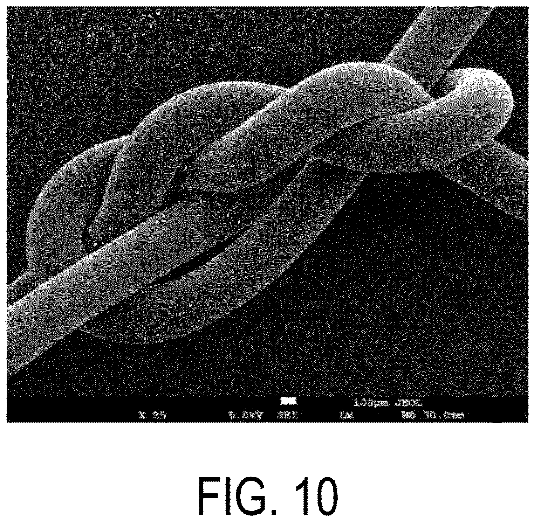

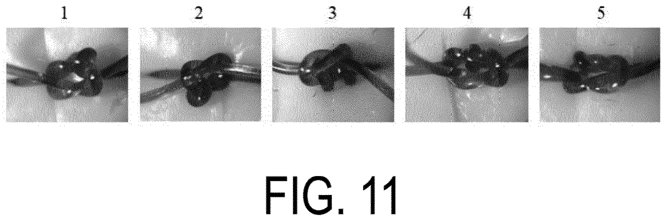

The same procedures were performed as in Example 2 except that a MonoMax suture (2-0 size) made of P(4HB) (available from BRAUN) to perform scanning electron microscopic observation of the suture surface (FIG. 9), scanning electron microscopic observation of the surgical knot (FIG. 10), and analysis of the circumference of the knot and the area surrounded by the circumference (FIG. 11), and the area surrounded by circumference/yarn diameter was calculated. The results are shown in Table 5 (Comparative Example 1).

The average value of the yarn diameter of the MonoMax suture (2-0 size) made of P(4HB) in Comparative Example 1 was 0.346 mm, which was certainly a yarn diameter corresponding to 2-0 according to the USP standard. The knot size of the surgical knot fastened with a force of 5 N was 6.91 mm for the average perimeter and 2.60 mm$^2$ for the average area of the region surrounded by the perimeter. A series of optical microscope images of the surgical knot used for the calculation is shown in FIG. 11.

The average value of the [area surrounded by perimeter/yarn diameter] as the index of the knot size was 7.50, which was clearly larger than those in Examples 1 to 4.

[Table 5]

| | Material, USP standard | Test No. | Yarn diameter (mm) | Perimeter*1 (mm) | Area surrounded by perimeter*2 (mm2) | Area surrounded by perimeter/yarn diameter |
|---|---|---|---|---|---|---|
| Comparative Example 1 | P(4HB) 2-0 size | B1-1 | 0.348 | 6.98 | 2.75 | 7.90 |
| | | B1-2 | 0.348 | 6.35 | 2.30 | 6.61 |
| | | B1-3 | 0.347 | 7.16 | 2.31 | 6.65 |
| | | B1-4 | 0.344 | 7.07 | 2.89 | 8.41 |
| | | B1-5 | 0.345 | 6.98 | 2.74 | 7.93 |
| | | Average | 0.346 | 6.91 | 2.60 | 7.50 |
| *1 Perimeter of outline of knot when viewed from one direction *2 Area of region surrounded by perimeter of outline of knot when viewed from one direction | | | | | | |

<Comparative Example 2> PDSII suture (3-0 size)

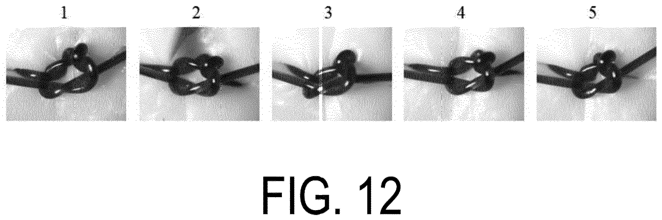

The same procedures were performed as in Comparative Example 1 except that an ETHICON PDSII suture (3-0 size) made of polydioxanone was used and that the scanning electron microscopic observation of the suture surface and the knot was omitted to analyze the yarn diameter, the perimeter of the knot, and the area surrounded by the perimeter (FIG. 12), and the area surrounded by the perimeter/yarn diameter was calculated. The results are shown in Table 6.

The average value of the yarn diameter of the PDSII suture (3-0 size) made of polydioxanone in Comparative Example 2 is 0.291 mm, but the medical device package insert states that the PDS suture falls within the USP standard except for the diameter, that the upper limit of the standard value of the yarn diameter is set to be larger than that according to the USP, and that the yarn diameter is larger than the standard value by 0.056 mm at the maximum in the case of 3-0 size. Thus, subtracting 0.056 mm from 0.291 mm gives 0.235 mm, which corresponded to the USP 3-0 size, but the actual yarn diameter was 0.291 mm on average. The knot size of the surgical knot fastened with a force of 5 N was 6.48 mm for the average perimeter and 2.28 mm$^2$ for the average area of the plane surrounded by the perimeter. A series of optical microscope images of the surgical knot used for the calculation is shown in FIG. 12.

The average value of the [area surrounded by perimeter/yarn diameter] as the index of the knot size of the PDSII suture used in Comparative Example 2 was 7.82, which was clearly larger than that of the P(3HB-co-4HB) sutures of Examples 1 to 4, but was almost equivalent to that of the MonoMax suture which was the P(4HB) suture of Comparative Example 1.

[Table 6]

| | Material, USP standard | Test No. | Yarn diameter (mm) | Perimeter *1 (mm) | Area surrounded by perimeter*2 (mm2) | Area surrounded by perimeter/yarn diameter |
|---|---|---|---|---|---|---|
| Comparative Example 2 | PDS 3-0 size*3 | B2-1 | 0.290 | 7.20 | 2.72 | 9.39 |
| | | B2-2 | 0.294 | 6.42 | 2.37 | 8.05 |
| | | B2-3 | 0.293 | 6.33 | 2.02 | 6.89 |
| | | B2-4 | 0.287 | 6.24 | 2.20 | 7.65 |
| | | B2-5 | 0.292 | 6.22 | 2.09 | 7.14 |
| | | Average | 0.291 | 6.48 | 2.28 | 7.82 |

*1 Perimeter of outline of knot when viewed from one direction
*2 Area of region surrounded by perimeter of outline of knot when viewed from one direction
*3 The medical device package insert states that the diameter is 0.056 mm larger than the USP standard value at the maximum.

<Comparative Example 3> PDSII suture (4-0 size)

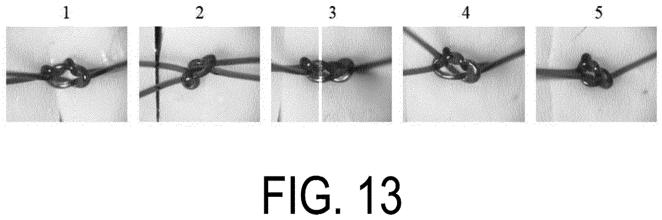

The same procedures were performed as in Example 2 except that an ETHICON PDSII suture (4-0 size) made of polydioxanone was used and that the scanning electron microscopic observation of the suture surface and the knot was omitted to analyze the yarn diameter, the perimeter of the knot, and the area surrounded by the perimeter (FIG. 13), and the area surrounded by the perimeter/yarn diameter was calculated. The results are shown in Table 7.

The average value of the yarn diameter of the PDSII suture (4-0 size) made of polydioxanone in Comparative Example 3 is 0.163 mm, but the medical device package insert states that the PDS suture falls within the USP standard except for the diameter, that the upper limit of the standard value of the yarn diameter is set to be larger than that according to the USP, and that the yarn diameter is larger than the standard value by 0.029 mm at the maximum in the case of 4-0 size. The actually measured yarn diameter of the suture of Comparative Example 3 was 0.163 mm, which was within the USP 4-0 size. The knot size of the surgical knot fastened with a force of 5 N was 4.77 mm for the average perimeter and 1.20 mm$^2$ for the average area of the plane surrounded by the perimeter. A series of optical microscope images of the surgical knot used for the calculation is shown in FIG. 13.

The average value of the [area surrounded by perimeter/yarn diameter] as the index of the knot size of the PDSII suture used in Comparative Example 3 was 7.33, which was clearly larger than that of the P(3HB-co-4HB) sutures of Examples 1 to 4, but was almost equivalent to those of the MonoMax suture which was the P(4HB) suture of Comparative Example 1 (2-0 size) and the PDSII suture of Comparative Example 2 (3-0 size).

[Table 7]

| | Material, USP standard | Test No. | Yarn diameter (mm) | Perimeter*1 (mm) | Area surrounded by perimeter*2 (mm2) | Area surrounded by perimeter/yarn diameter |
|---|---|---|---|---|---|---|
| Comparative Example 3 | PDS 4-0 size | B3-1 | 0.165 | 4.96 | 1.32 | 8.01 |
| | | B3-2 | 0.161 | 4.24 | 0.917 | 5.70 |
| | | B3-3 | 0.165 | 5.30 | 1.35 | 8.16 |
| | | B3-4 | 0.163 | 5.10 | 1.40 | 8.60 |
| | | B3-5 | 0.162 | 4.24 | 0.993 | 6.13 |
| | | Average | 0.163 | 4.77 | 1.20 | 7.33 |

*1 Perimeter of outline of knot when viewed from one direction
*2 Area of region surrounded by perimeter of outline of knot when viewed from one direction

Based on a comparison among the knot perimeters of the surgical knots, the facts that the perimeter of 4.86 mm of the knot of the P(3HB-co-4 HB) suture of 1 size (average yarn diameter: 0.406 mm) in Example 3 is smaller than the knot perimeter of 6.91 mm of the MonoMax suture 2-0 size (average yarn diameter: 0.346 mm) of Comparative Example 1 and the knot perimeter of 6.48 mm of the PDSII suture 3-0 size (average yarn diameter: 0.291 mm) of Comparative Example 2, and further is equivalent to the knot perimeter of 4.77 mm of the PDSII suture 4-0 size (average yarn diameter: 0.163 mm) of Comparative Example 3 indicate that the knot of the P(3HB-co-4 HB) suture as a stretchable suture is smaller than those of the other absorbent sutures. As described above, from the results of Examples 1 to 4 and Comparative Examples 1 to 3, it was suggested that the knot of the P(3HB-co-4HB) suture is smaller than the knot of the MonoMax suture or the PDSII suture as the existing absorbable monofilament suture.

<Comparison in Knot Security Factor (Knot Security)>

The difficulty in unraveling (knot security) of a ligated part was evaluated in vitro using a Knot Security factor (KSF) described also in Odermatt et al. (International Journal of Polymer Science, Vol.2012, Article ID216137).

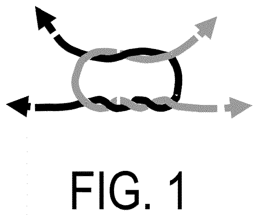

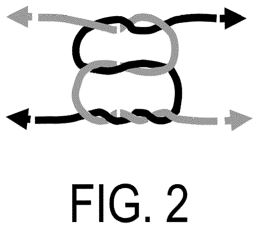

The suture was wound around a plastic tube having a diameter of 2.9 cm, tied tightly to form a surgical knot (FIG. 1), and cut on the side opposite to the knot to prepare a single suture. Both sides of the suture were attached to a tensile tester, and the suture was pulled at a speed of 100 mm/min. A plurality of sets of 10 samples were provided, and, if even at least one of the 10 samples unraveled at the Knot part, a single knot was added onto the surgical knot (FIG. 2). Single knots were added until there was no longer sample which unraveled at the Knot part. The number of single knots added until all the 10 samples did not unravel at the Knot part was defined as Knot Security factor (KSF).

Thus, KSF can be exemplified as follows.

KSF = 0 The sample does not unravel at the knot under tension, with only a surgical knot, and has no additional single knot.
KSF = 1 The sample does not unravel under tension, with only one single knot added onto the surgical knot.
KSF = 2 The sample does not unravel under tension, with only two single knots added onto the surgical knot.
KSF = 3 The sample does not unravel under tension, with only three single knots added onto the surgical knot.
KSF = 4 The sample does not unravel under tension, with only four single knots added onto the surgical knot.
KSF = n The sample does not unravel under tension, with only n single knots added onto the surgical knot.

In the case of monofilament yarns, it can be generally said that excellent knot security is given when $2 \leq KSF \leq 4$, and that absolutely excellent knot security is given when $KSF \leq 1$.

The KSFs of the P(3HB-co-14.8 mol% 4HB) stretchable monofilament yarns including the P(3HB-co-14.8 mol% 4HB) yarn shown in Example 1 were evaluated as 1, because all the stretchable yarns having thicknesses corresponding to the average diameters 3-0, 2-0, and 0 do not unravel under tension, with only one single knot added onto the surgical knot. Thus, the yarns can be judged as having absolutely excellent knot security.

Both the KSFs of the P(3HB-co-15.3 mol% 4HB) stretchable monofilament yarn (3-0 size) indicated in Example 2 and the P(3HB-co-15.3 mol% 4HB) stretch monofilament yarn (3-0 size) indicated in Example 3 were evaluated as 2, because both the yarns do not unravel under tension, with only two single knots added onto the surgical knot. Thus, the yarns can be judged as having excellent knot security.

The KSF of the P(3HB-co-16.0 mol% 4HB) stretchable monofilament yarn (2.5-0 size) indicated in Example 4 was evaluated as 2 as in Examples 2 and 3, because the yarn does not unravel under tension, with only two single knots added onto the surgical knot. Thus, the yarn can be judged as having excellent knot security.

The KSFs of the MonoMax sutures (3-0, 2-0 and 0 standards) made of P(4HB) shown in Comparative Example 1 were evaluated as 2, 2 and 3, respectively (International Journal of Polymer Science, Vol.2012, Article ID216137).

The KSFs of the PDSII sutures (3-0 and 4-0) and PDSII suture (2-0) made of polydioxanone, which are shown in Comparative Examples 2 and 3, were evaluated as 3, because all the yarns do not unravel under tension, with three single knots added onto the surgical knot. Thus, the yarns can be judged as having excellent knot security.

<Comparative Examples 4 and 5>

When the data of Silver E et al. (J.Oral.Maxillofac.Surg.2016 Jul; 74(7): 1304-1312) is replaced with KSF, the KSFs of the nylon sutures (Comparative Example 4) 3-0 and 4-0 would be both evaluated as 3, and the KSFs of the multifilament Vicryl sutures (Comparative Example 5) 3-0 and 4-0 are evaluated as 4 and 3, respectively. These KSF values are shown in Table 8.

[Table 8]

|  | USP yarn diameter | KSF[*2] |
|---|---|---|
| P(3HB-co-14.8 mol% 4HB)(monofilament) | 0[*1] | 1 |
| P(3HB-co-14.8 mol% 4HB)(monofilament) | 2-0[*1] | 1 |
| P(3HB-co-14.8 mol% 4HB)(monofilament) | 2.5-0[*1] (Example 1) | 1 |
| P(3HB-co-15.3 mol% 4HB) (monofilament) | 3-0 (Example 2) | 2 |
| P(3HB-co-15.3 mol% 4HB) (monofilament) | 3-0 (Example 3) | 2 |
| P(3HB-co-16.0 mol% 4HB) (monofilament) | 2.5-0 (Example 4) | 2 |
| MonoMax (P(4HB)) (monofilament) | 0 | 3 |
| MonoMax (P(4HB)) (monofilament) | 2-0 (Comparative Example 1) | 2 |
| MonoMax (P(4HB)) (monofilament) | 3-0 | 2 |
| PDSII (polydioxanone) (monofilament) | 2-0 | 3 |
| PDSII (polydioxanone) (monofilament) | 3-0 (Comparative Example 2) | 3 |
| PDSII (polydioxanone) (monofilament) | 4-0 (Comparative Example 3) | 3 |
| Nylon (monofilament) Comparative Example 4 | 3-0 | 3 |
| Nylon (monofilament) Comparative Example 4 | 4-0 | 3 |
| Vicryl (Braid) Comparative Example 5 | 3-0 | 4 |
| Vicryl (Braid) Comparative Example 5 | 4-0 | 3 |

*1 Size when average diameter between short-direction yarn diameter (minor axis length) and long-direction yarn diameter (major axis length) is converted into USP size
*2 Knot security factor

As described above, the KSFs of the P(3HB-co-4HB) stretchable monofilament sutures were better than those of the other sutures such as the other bioabsorbable/non-absorbable monofilament sutures and bioabsorbable multifilament (braid) sutures. In the case of the P(3HB-co-4HB) stretchable monofilament suture, the number of single knots to be added to the surgical knot can be made smaller than that for the other sutures with the fear that the knot may unravel after surgery, and thus the knot formed at the time of actual surgery is expected to contribute to the reduction in foreign-body sensation given to the surrounding tissue in which the knot is embedded, because the volume occupied by the knot itself does not increase due to the effect of reducing the number of knots themselves and the effect of forming a small knot by the stretching of the suture itself. In addition, the P(3HB-co-4HB) stretchable suture has a low initial elastic modulus in tension and an intermediate elastic modulus in tension lower than the initial elastic modulus in tension even if it is a monofilament, and is pliable, and when a knot is formed, it is easily and firmly tightened without applying an excessive force and does not immediately start to loosen, so that it has very good operability. Further, since the previously tied knot is not loosened, the single knot addition is easy. Other monofilament sutures (existing monofilament sutures such as MonoMax, PDSII, and nylon) are stiff and impliable, still retain a high intermediate elastic modulus in tension above 400 MPa, are incompliant as compared with the P(3HB-co-4HB) sutures, and are often pulled with excessive force to firmly tighten the knot for preventing the knot from coming loose from as soon as the knot is formed. In such a situation, when the suture forming a knot is tightened with a strong force, an excessive force is applied to sutured part tissues, leading to a risk that the tissue may be unintentionally damaged, and concern about an influence on the tissue. A site requiring ligation during surgery is not necessarily a site with a free and wide space, and, in some cases, there are many situations requiring ligation in a narrow range of motion or in a narrow surgical field. In such cases, it is also true that there is a need for a suture with good operability, which allows formation of a firm knot with a light force. A suture satisfying the knot tensile strength as specified in the current USP standard has a high elastic modulus, which is not necessarily satisfactory from the viewpoint of ease of knot formation and ease of knot loosening. Therefore, there is a need in medical practice for a suture that has lower elastic moduli (initial elastic modulus in tension and intermediate elastic modulus in tension) than those of existing sutures, has high followability to tissue deformation, and has good operability.

Due to a small and hard-to-unravel knot, it is expected that, when a surplus suture is cut off from the knot, the need to leave a cut end of the suture long is reduced, and that, in the case of a flexible suture, tingling irritation to tissue caused by a stiff cut end of the suture is also reduced, and this is also expected to contribute to the reduction in foreign-body sensation given to the surrounding tissue.

Young's moduli of the respective tissues of the living tissue are summarized in Funai et al. (Reports of the Industrial Research Institute of Shizuoka Prefecture, 2007, No. 52, pp.33-37 "Creation of physical property value database of biological tissue for biomechanical simulation and application thereof"). While elastic moduli of teeth and sebaceous bones are high, i.e., greater than 10000 MPa, elastic modulus of ligaments is 248 MPa, elastic modulus of cartilage is 23 MPa, elastic modulus of corneas is 20 MPa, and elastic moduli of various internal organs, muscles, skin and other soft tissues are 10 MPa or less. Even the lowest elastic modulus among existing absorbable sutures is 485 MPa for MonoMax, and there is no existing absorbable suture having an elastic modulus close to that of soft tissue.

<Ethylene Oxide Gas (EOG) Sterilization of PHA Stretchable Suture>

The P(3HB-co-4HB) suture of Example 1 was subjected to EO gas sterilization. First, the P(3HB-co-4HB) suture cut into an appropriate length was wrapped in a sterilization wrapping material having ethylene oxide gas permeability (hybrid sterilization bag HM-1304: available from HOGY MEDICAL CO., LTD), and an opening was heated and sealed using a heat sealer. The P(3HB-co-4HB) stretchable suture wrapped in the sterilization wrapping material was sterilized with ethylene oxide gas at 40°C for 5 hours with 15 g of 95% ethylene oxide (EKITEC 95, available from NIPPON EKITAN Corporation) using a total automatic ethylene oxide gas sterilizer (Eogelk, SA-N160, available from ELK Corporation), air substitution was performed for 2 hours, and then an aerating treatment was performed for 14 hours.

No obvious macroscopic or microscopic structural or physical changes were observed in the P(3HB-co-4HB) suture.

<In Vitro Decomposition of P(3HB-co-4HB) Stretchable Suture>

In order to evaluate the decomposability of the P(3HB-co-4HB) stretchable suture, the decomposition behavior in a buffer was evaluated in vitro. The method will be indicated below.

The EOG-sterilized PHA suture having 3-0 size of Example 1 was immersed in Dulbecco's phosphate buffer (pH 7.4; 37°C), taken out after 1, 2, 3, 4, 6, 8, 12 and 16 weeks, lightly washed with water, vacuum dried, and subjected to a tensile test and molecular weight measurement. Those not immersed in the acid buffer were designated as 0 weeks (initial).

The P(3HB-co-4HB) stretchable suture was subjected to a tensile test to break by using a drawn PHA fiber having a length of 3 cm and a fiber diameter of from approximately 0.1 to 0.3 mm and a tensile tester AGS-50NX (available from Shimadzu Corporation) under conditions of a temperature of 23°C, a test speed of 10 mm/min, and an initial length (chuck-to-chuck distance) of 10 mm.

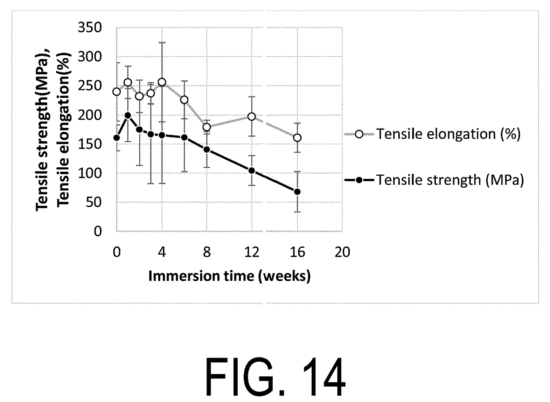

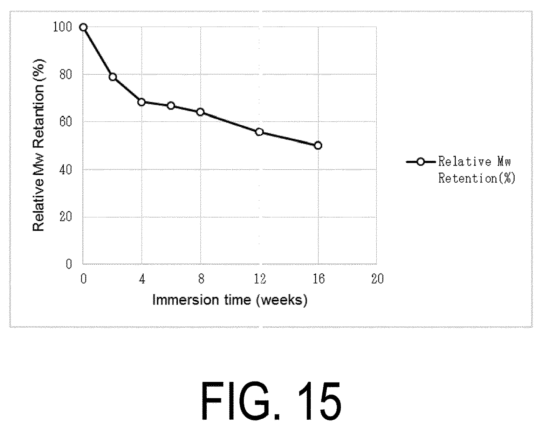

The results of the tensile test are shown in FIG. 14, and the degree of decrease in molecular weight during the immersion period is shown in FIG. 15, with the weight average molecular weight Mw of 320,000 of the sample before immersion being taken as 100%.

An initial fracture elongation of the P(3HB-co-4HB) stretchable suture was greater than 180% as also shown in FIG. 14.

When the PHA stretchable suture was immersed in the buffer, the period during which half of an initial linear tensile fracture strength could be maintained was approximately 16 weeks (FIG. 14), and the weight average molecular weight was also halved in approximately 16 weeks (FIG. 15). The fracture elongation was also maintained at 150% on average even after 16 weeks (FIG. 14), and it was found that the characteristic of high stretchability of this suture was maintained.

According to Williams et al. (Biomed Tech (Berl). 2013 Oct; 58 (5): 439 to 452, Poly-4-hydroxybutyrate (P4HB): a new generation of resorbable medical devices for tissue repair and regeneration), it has been reported that an oriented P(4HB) fiber has a fracture elongation of approximately from 25 to 90% or less, and that a Monomax suture having a diameter of 0.154 mm and composed of an oriented P(4HB) has a fracture elongation of 35%. Albertsmeier et al. (Langenbecks Arch Surg (2012) 397: 363-371, Evaluation of the safety and efficacy of MonoMax[R] suture material for abdominal wall closure after primary midline laparotomy-a controlled prospective multicentre trial: ISSAAC [NCT005725079]) describes that a MonoMax suture has a fracture elongation of 90%, and that a PDS suture or MonoPlus suture made of polydioxanone has a fracture elongation of from 45 to 50%. The P(3HB-co-4HB) stretchable suture obviously has a fracture elongation approximately from 2 to 5 times larger than those of these sutures, and is expected to have a property of being able to follow tissue deformation.

<In Vivo Decomposition of P(3HB-co-4HB) Stretchable Suture>

In order to evaluate the decomposability of the P(3HB-co-4HB) stretchable suture, the in vivo decomposition behavior was evaluated in a rat body (in vivo). The method will be indicated below.

The dorsal skin of a rat (F344/NSlc male, 20 weeks old) was incised by 8 cm along the spinal column, and the EOG-sterilized PHA suture of Example 1 was implanted into the subcutaneous tissue. After 4, 8, 12, 16, and 26 weeks, samples were collected, lightly washed with water, vacuum dried, and subjected to a tensile test and molecular weight measurement. Those not implanted were designated as 0 weeks (initial).

The P(3HB-co-4HB) stretchable suture was subjected to a tensile test to break by using a drawn PHA fiber having a length of 3 cm and a fiber diameter of from approximately 0.1 to 0.3 mm and a tensile tester AGS-50NX (available from Shimadzu Corporation) under conditions of a temperature of 23°C, a test speed of 10 mm/min, and an initial length (chuck-to-chuck distance) of 10 mm.

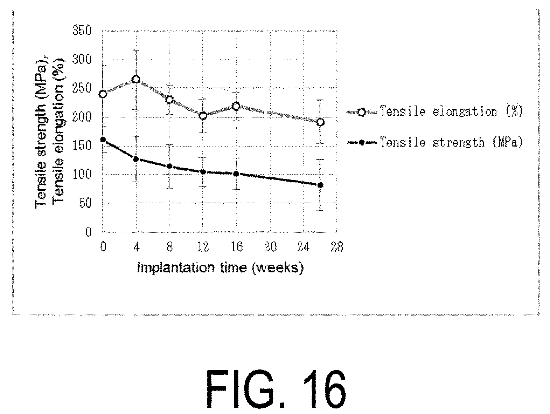

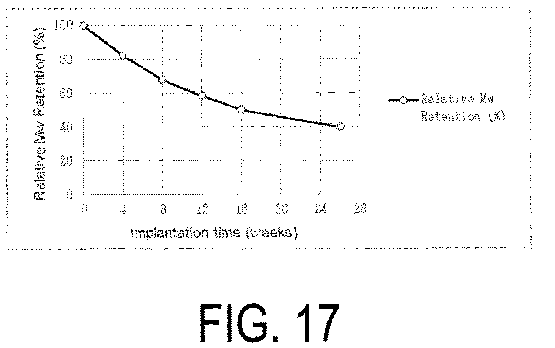

The results of the tensile test are shown in FIG. 16, and the degree of decrease in molecular weight during the implantation period is shown in FIG. 17, with the weight average molecular weight of the sample before implantation being taken as 100%.

When the PHA stretchable suture was implanted into the rat subcutaneous tissue, the period during which half of the initial linear tensile fracture strength could be maintained was approximately from 16 weeks to 24 weeks (FIG. 16), and the weight average molecular weight was also halved in approximately 16 weeks (FIG. 17). The fracture elongation was also maintained at 150% even after 26 weeks (FIG. 16).

It has been shown that the in vivo tensile fracture strength of the MonoMax suture 3-0 made of P(4HB) can maintain half of the initial fracture strength for 12 weeks, and that the in vivo tensile fracture strength of PDSII 3-0 made of polydioxanone was halved in 6 weeks (International Journal of Polymer Science, Vol.2012, Article ID216137).

From these facts, it is conceivable that the P(3HB-co-4HB) stretchable suture can be used at a site where tensile strength is desired to be retained for a longer period than the MonoMax suture or the PDSII suture.

<Microminipig Abdominal Wall Suture Test>

With the P(3HB-co-4HB) stretchable suture of Example 1 and other absorbable sutures, a 32-week-old female microminipig (Fuji Micra Inc.) was subjected to a suture test for the purpose of macroscopically and microscopically evaluating the presence or absence of complications and the amount of inflammatory reaction.

After a fetus was taken out by Cesarean section, the abdominal wall was sutured by 12 cm with three stitches with head-side P(3HB-co-4HB) sutures, two stitches with central poly(glycolide-co-trimethylene carbonate) (PGA-TMC copolymer) sutures (Maxon sutures), and three stitches with tail-side P(4HB) sutures (MonoMax sutures).

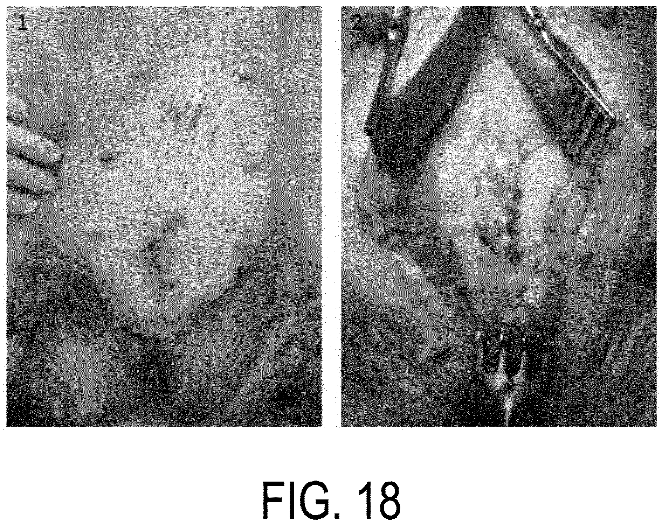

At 7 weeks after the suture, the presence or absence of complications (signs of infection, wound dehiscence, abdominal incisional hernia, and synechia) in the sutured parts was macroscopically observed, but no remarkable complications were observed in any of the sutured parts with the three types of sutures (FIG. 18).

Then, the abdominal wall including the sutured parts was collected, and the sutured parts of the respective sutures were cut and separated, and each fixed with paraffin, followed by hematoxylin-eosin (HE) staining according to a routine method, and the amount of inflammation was observed with an optical microscope. In the tissue observation, one pathologist evaluated inflammation, necrosis, and fibrous thickening, on a four-level scale of 0, 1, 2, and 3, by a blind method in which the correspondence between sample and suture was not clarified.

0: No inflammatory reaction, no necrosis, and no thickening
1: Very little inflammatory reaction, very little necrosis, and very little thickening
2: Inflammatory reaction, necrosis, and thickening
3: Strong inflammatory reaction, strong necrosis, and strong thickening

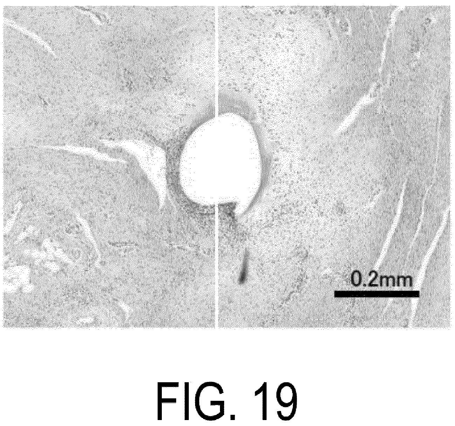

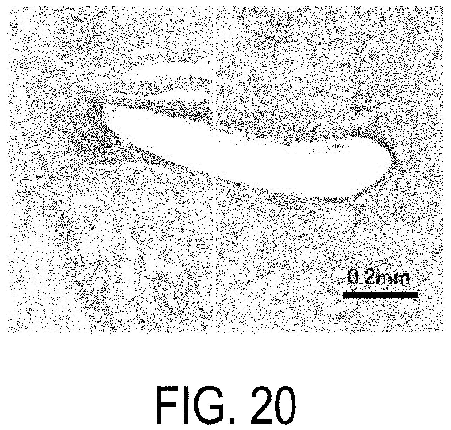

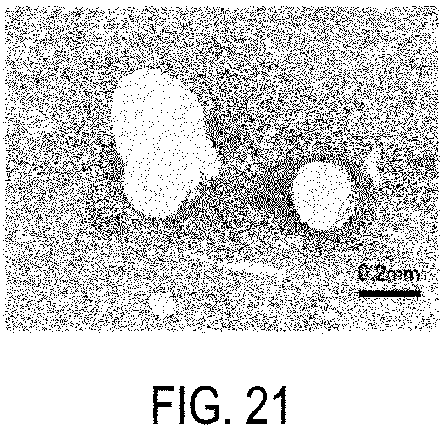

Two weeks after the initial observation, re-evaluation was performed. When the first and second evaluations were different, re-evaluation (third) was performed again two weeks later for final determination. The results are shown in Table 9. FIGS. 19, 20 and 21 are images showing the results of HE staining of the tissues sutured with the P(3HB-co-4HB) suture, the poly (glycolide-co-trimethylene carbonate) (PGA-TMC) suture and the P(4HB) suture, respectively.

As a result of tissue observation, it was suggested that the P(3HB-co-4HB) suture caused less inflammation as compared with the other two types of sutures. There was also a possibility of the P(3HB-co-4HB) suture was not inferior in terms of necrosis and fibrous thickening.

[Table 9]

|  | P(3HB-co-4HB) | PGA-TMC | P(4HB) |
|---|---|---|---|
| Inflammation [1) | 1 | 2 | 3 |
| Necrosis [1) | 1 | 1 | 1 |

(continued)

|  | P(3HB-co-4HB) | PGA-TMC | P(4HB) |
|---|---|---|---|
| Fibrous thickening [1] | 1 | 1 | 2 |
| 1) Evaluated by the blind method performed by the pathologist. | | | |

The characteristics of causing less inflammation and potentially not inferior also in terms of necrosis and fibrous thickening help demonstrate the utility of the P(3HB-co-4HB) suture. Also in consideration of the characteristic that the knot in the sutured part becomes small, it has been demonstrated that the P(3HB-co-4HB) absorbable stretchable suture, which is long-term absorbable and stretchable, and significant as a suture that makes the knot itself smaller, is an attractive medical device having a new potential for application, as a suture which can be applied to a site where known sutures cannot be used due to too rapid decomposition and absorption in the past medical care or in the case where soft tissue is damaged because of too strong tension of sutures as compared with the tissue or difficulty in elongation.

<Tensile Break Evaluation of P(3HB-co-4HB) Stretchable Suture>

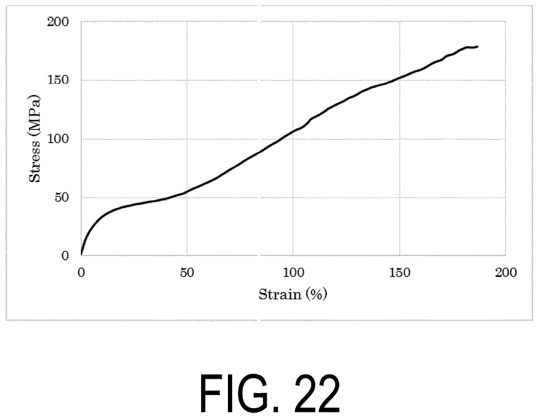

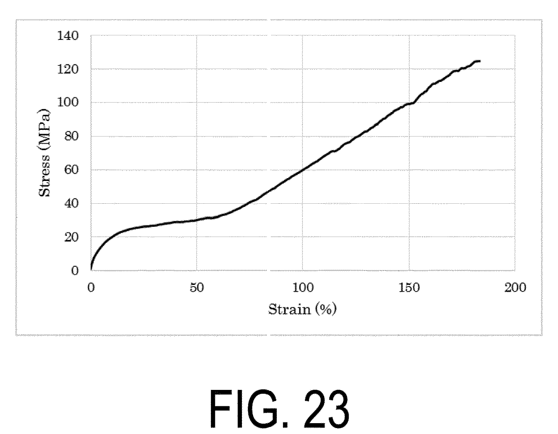

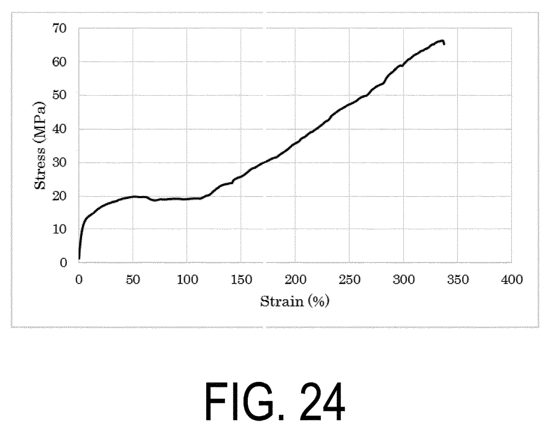

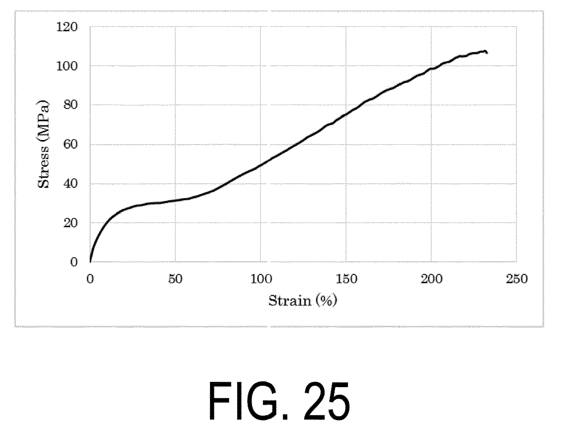

The same yarn as the P(3HB-co-4HB) stretchable suture used in Example 1 was evaluated again by a tensile test in which it was pulled to break. A P(3HB-co-4 HB) suture having a length of 3 cm, a minor-axis average fiber diameter of approximately 0.24 mm, and a major-axis average fiber diameter of approximately 0.40 mm (major axis length/minor axis length = 1.7) was subjected to a tensile test to break of the fiber under the conditions of a temperature of 23°C, a test speed of 10 mm/min, and an initial length (chuck-to-chuck distance) of 10 mm using a tensile tester AGS-50 NX (available from Shimadzu Corporation). An example of the result of stress-strain curve is shown in FIG. 22. Similarly, examples of the results of stress-strain curves of tensile tests to break in Examples 2 to 4 are shown in FIGS. 23 to 25.
As an example, the tensile fracture strength of the fiber used in Example 1 and also shown in FIG. 22 was 161 MPa as an average for five points, and the fracture elongation thereof was 240% (variation: from 180 to 282%).
In addition, the tensile fracture strength of the fiber used in Example 2 and also shown in FIG. 23 was 120 MPa as an average for five points, and the fracture elongation thereof was 183% (variation: from 157 to 209%).
The tensile fracture strength of the fiber used in Example 3 and also shown in FIG. 24 was 69 MPa as an average for five points, and the fracture elongation thereof was 250% (variation: from 178 to 338%).
The tensile fracture strength of the fiber used in Example 4 and also shown in FIG. 25 was 110 MPa as an average for five points, and the fracture elongation thereof was 232% (variation: from 192 to 272%).

<Evaluation of Stretch Recovery and Residual Strain of P(3HB-co-4HB) Stretchable Suture>

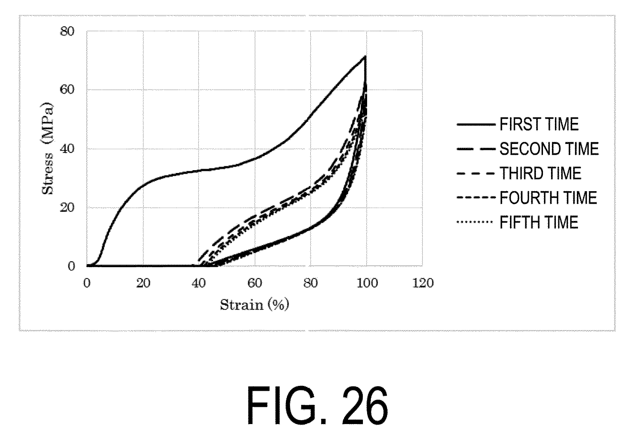

The P(3HB-co-4HB) stretchable suture used in Example 1 was evaluated by a cycle test in which it was repeatedly stretched. The P(3HB-co-4HB) suture having a length of 3 cm and a major-axis thickness fiber diameter of approximately 0.2 mm was subjected to a cycle test by using a tensile tester AGS-50NX (available from Shimadzu Corporation) under the conditions of a temperature of 23°C and an initial length of 10 mm. Stretching was performed at a tensile speed of 20 mm/min to a strain of 100% (2-fold length), then the gripper was moved to the original length at the same speed to allow the PHA fiber to shrink. This was repeated five times. The stress-strain curves at the time of the first to fifth shrinkages were shown in FIG. 26.
The P(3HB-co-4HB) stretchable suture used in this Example 1 has a tensile elongation recovery rate (%) of approximately 60% and a residual strain rate of approximately 40% at the beginning of the second elongation (i.e., considered to be approximately equal to the end of the first shrinkage) when subjected to 100% strain during elongation. At the beginning of the third to fifth elongations, the tensile elongation recovery rate (%) was from approximately 60% to approximately 55%, and the residual strain rate was from approximately 40% to approximately 45% (FIG. 26).
In the case where the following operations are repeated: conducting a cycle test of a fiber having a length 3 cm using a tensile tester under conditions of a temperature of 23°C and an initial length of 10 mm, stretching the fiber at a tensile speed of 20 mm/min to a strain of 100% (20 mm which is a length 2 fold the initial length, i.e., a displacement length of 10 mm), subsequently moving a gripper to the original length at the same speed, and shrinking the fiber,

a tensile elongation recovery rate $R_{100}$(%) is expressed as follows:

$$R_{100} = [20 - (X_{100} + 10)]/10 \times 100,$$

when a displacement length at the beginning of the second elongation (i.e., considered to be approximately equal to the end of the first shrinkage) is $X_{100}$ mm.

A residual strain rate $S_{100}$ (%) is expressed as follows:

$$S_{100} = 100 - R_{100}.$$

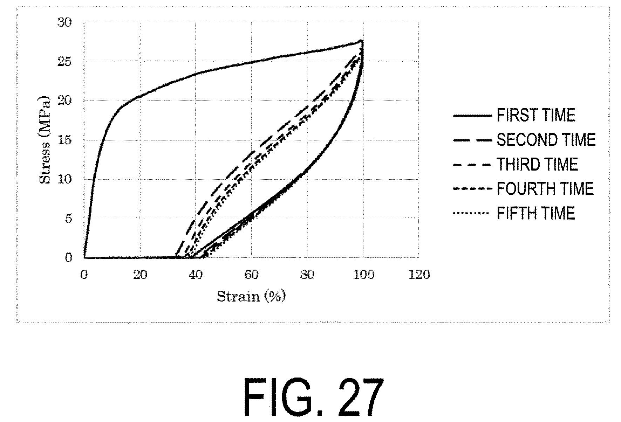

The P(3HB-co-4HB) stretchable suture used in Example 2 was evaluated by a cycle test in which it was repeatedly stretched. The P(3HB-co-4HB) suture having a length of 3 cm and a fiber diameter of approximately 0.207 mm was subjected to a cycle test by using a tensile tester AGS-50NX (available from Shimadzu Corporation) under the conditions of a temperature of 23°C and an initial length of 10 mm. Stretching was performed at a tensile speed of 20 mm/min to a strain of 100% (2-fold length), then the gripper was moved to the original length at the same speed to allow the PHA fiber to shrink. This was repeated five times. The stress-strain curves at the time of the first to fifth shrinkages were shown in FIG. 27.

The P(3HB-co-4HB) stretchable suture used in this Example 2 has a tensile elongation recovery rate (%) of approximately 67% and a residual strain rate of approximately 33% at the beginning of the second elongation (i.e., considered to be approximately equal to the end of the first shrinkage) when subjected to 100% strain during elongation. At the beginning of the third to fifth elongations, the tensile elongation recovery rate (%) was from approximately 63% to approximately 60%, and the residual strain rate was from approximately 37% to approximately 40% (FIG. 27).

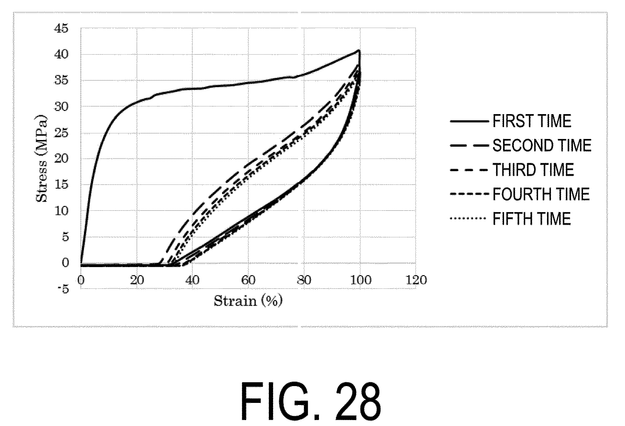

The P(3HB-co-4HB) stretchable suture used in Example 3 was evaluated by a cycle test in which it was repeatedly stretched. The P(3HB-co-4HB) suture having a length of 3 cm and a fiber diameter of approximately 0.410 mm was subjected to a cycle test by using a tensile tester AGS-50NX (available from Shimadzu Corporation) under the conditions of a temperature of 23°C and an initial length of 10 mm. Stretching was performed at a tensile speed of 20 mm/min to a strain of 100% (2-fold length), then the gripper was moved to the original length at the same speed to allow the PHA fiber to shrink. This was repeated five times. The stress-strain curves at the time of the first to fifth shrinkages were shown in FIG. 28.

The P(3HB-co-4HB) stretchable suture used in this Example 3 has a tensile elongation recovery rate (%) of approximately 70% and a residual strain rate of approximately 30% at the beginning of the second elongation (i.e., considered to be approximately equal to the end of the first shrinkage) when subjected to 100% strain during elongation. At the beginning of the third to fifth elongations, the tensile elongation recovery rate (%) was from approximately 63% to approximately 68%, and the residual strain rate was from approximately 32% to approximately 37% (FIG. 28).

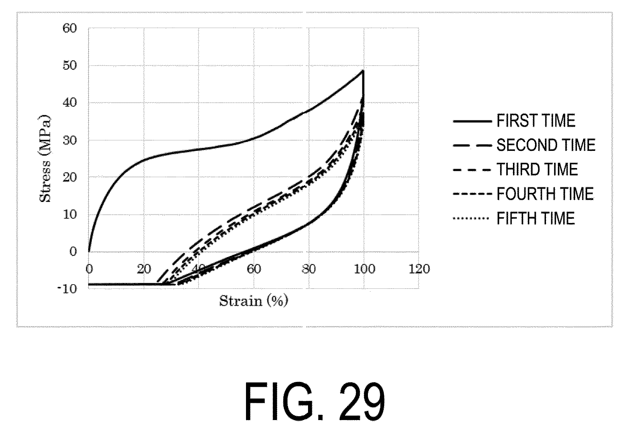

The P(3HB-co-4HB) stretchable suture used in Example 4 was evaluated by a cycle test in which it was repeatedly stretched. The P(3HB-co-4HB) suture having a length of 3 cm and a fiber diameter of approximately 277 mm was subjected to a cycle test by using a tensile tester AGS-50NX (available from Shimadzu Corporation) under the conditions of a temperature of 23°C and an initial length of 10 mm. Stretching was performed at a tensile speed of 20 mm/min to a strain of 100% (2-fold length), then the gripper was moved to the original length at the same speed to allow the PHA fiber to shrink. This was repeated five times. The stress-strain curves at the time of the first to fifth shrinkages were shown in FIG. 29.

The P(3HB-co-4HB) stretchable suture used in this Example 4 has a tensile elongation recovery rate (%) of approximately 74% and a residual strain rate of approximately 26% at the beginning of the second elongation (i.e., considered to be approximately equal to the end of the first shrinkage) when subjected to 100% strain during elongation. At the beginning of the third to fifth elongations, the tensile elongation recovery rate (%) was from approximately 72% to approximately 66%, and the residual strain rate was from approximately 28% to approximately 34% (FIG. 29).

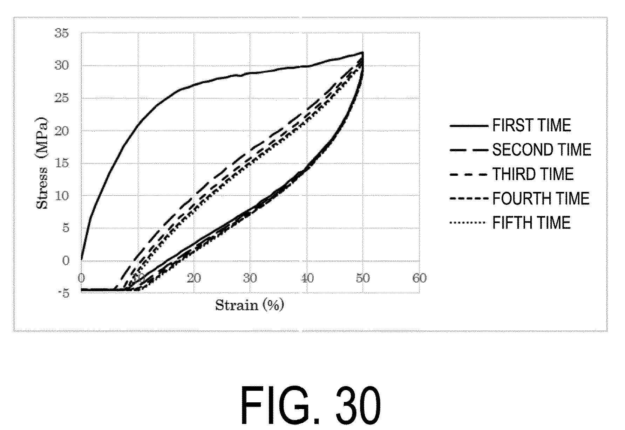

Further, the P(3HB-co-4HB) suture having a length of 12 cm and a fiber diameter of approximately 0.283 mm used in Example 4 was subjected to a cycle test by using a tensile tester AGS-50NX (available from Shimadzu Corporation) under the conditions of a temperature of 23°C and an initial length of 100 mm. Stretching was performed at a tensile speed of 100 mm/min to 50% strain (1.5-fold length), then the gripper was moved to the original length at the same speed to allow the PHA fiber to shrink. This was repeated five times. The stress-strain curves at the time of the first to fifth shrinkages were shown in FIG. 30.

The P(3HB-co-4HB) stretchable suture used in this Example 4 has a tensile elongation recovery rate (%) of approximately 94% and a residual strain rate of approximately 6% at the beginning of the second elongation (i.e., considered to be approximately equal to the end of the first shrinkage) after loading of 50% strain. At the beginning of the third to fifth elongations, the tensile elongation recovery rate (%) was from approximately 93% to approximately 90%, and the residual strain rate was from approximately 7% to approximately 10% (FIG. 30).

As described above, it has been revealed that the decrease in proportion of the initial strain decreases the residual strain, leading to a property of easy elastic recovery.

In the case where the following operations are repeated: conducting a cycle test of a fiber having a length 12 cm using a tensile tester under conditions of a temperature of 23°C and an initial length of 100 mm, stretching the fiber at a tensile speed of 100 mm/min to 50% strain (150 mm which is a length 1.5 fold the initial length, i.e., a displacement length of 50 mm), subsequently moving a gripper to the original length at the same speed, and shrinking the fiber,

a tensile elongation recovery rate $R_{50}$ (%) is expressed as follows:

$$R_{50} = [(150 - (X + 100))/50] \times 100,$$

when a displacement length at the beginning of second elongation (i.e., approximately equal to the end of first shrinkage) is $X_{50}$ mm.

A residual strain rate $S_{50}$ (%) is expressed as follows:

$$S_{50} = 100 - R_{50}.$$

<Cross Section Analysis of P(3HB-co-4HB) Stretchable Suture>

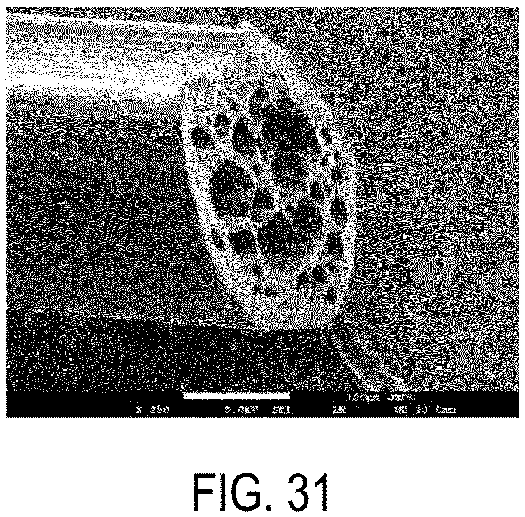

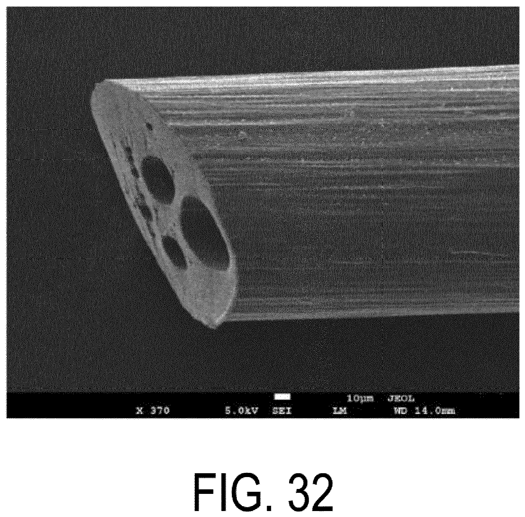

In order to explore the mechanism by which the knot of the P(3HB-co-4HB) stretchable suture becomes smaller, the cross section of the P(3HB-co-4HB) suture of Example 1 was observed with a scanning electron microscope. The results are shown in FIGS. 31 and 32. As shown in FIG. 3, the surface of the fiber was smooth and had no holes, but, as shown in FIGS. 31 and 32, the P(3HB-co-4HB) stretchable suture used in Example 1 had holes in the fiber. Image analysis revealed 43.5% gaps from the cross-sectional view of FIG. 31 and 24.9% gaps from the cross-sectional view of FIG. 32. Cross-sections of 10 sutures were measured, and the average porosity was $40 \pm 15\%$.

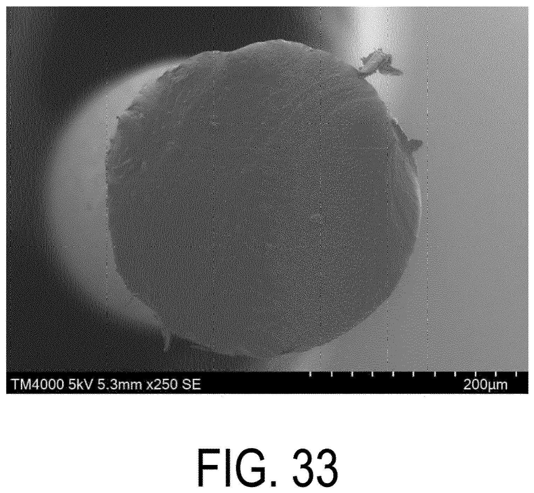

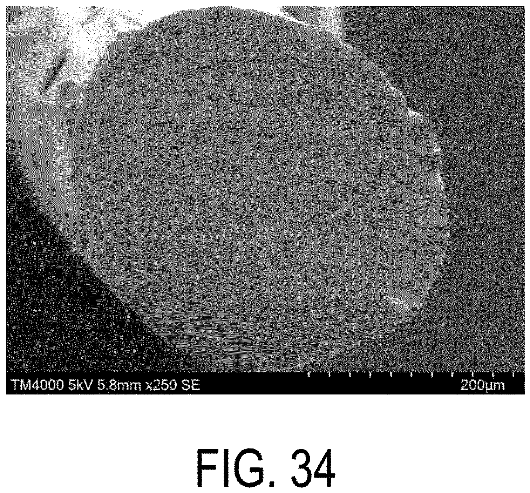

FIGS. 33 and 34 show scanning electron microscopic observations of the P(3HB-co-4HB) sutures used in Examples 2 and 3. Unlike FIGS. 31 and 32, it was observed that the cross sections shown in FIGS. 33 and 34 had no holes, and were densely packed, and this is a result supporting that the P(3HB-co-4HB) suture of Example 1 was opaque while the P(3HB-co-4HB) sutures of Examples 2 and 3 were colorless and transparent.

With respect to the change in polymer structure during stretching, observation has been performed, which suggests that an unstretched P(3HB-co-4HB) copolymer molded product retains an $\alpha$-form ($\alpha$ crystal) exhibiting random orientation; that the cycle of the $\alpha$ crystal is not uniform; but that, when the molded body is deformed by being stretched after a crystallization treatment for a certain time period, a degree of orientation of the $\alpha$ crystal is increased in a direction of the stretching, and, simultaneously, molecular chains of an amorphous part in between an $\alpha$ crystal and an $\alpha$ crystal are elongated to exhibit a $\beta$-form (plane zigzag structure); and that the $\beta$-form was reduced or eliminated by removal of load while the degree of orientation of the $\alpha$ crystal is maintained, to exhibit an elastic response, as described in Japanese Patent Application No. 2019-90739. Note that the $\alpha$-form is a folded lamellar structure, and the $\beta$-form is a plane zigzag stretched chain structure.

It was conceivable that the P(3HB-co-4HB) copolymer is not only a soft material with a low elastic modulus, but also can be spun and drawn into elastic a stretchable structure, and the presence of gaps in the fibers is also one of the factors that make the knots smaller.

There are various means for forming gaps, voids, fine pores, pores and the like in a non-bioabsorbable polymer, and a phase separation method, an extraction method, an electron beam irradiation/etching method, a polymer particle fusion method, a foaming agent mixing method, a gas mixing method, a drawing method and the like are known. For the formation of voids in a PHA fiber which is a bioabsorbable polymer, a microcrystalline nucleus drawing method is known, and impregnation of a drug solution into the inside of a PHA void fiber has been tried, but discovery that the presence of such gaps contributes to a small knot of a suture fiber is the first time and is a remarkable characteristic. The method for introducing voids into fibers is not particularly limited as long as fibers having strength can be provided.

<Discussion on Elastic Modulus>

The initial elastic moduli in tension and the intermediate elastic moduli in tension of the P(3HB-co-4HB) stretchable sutures of Examples 1 to 4 were measured using a tensile tester. Here, in the present specification, an elastic modulus calculated from a slope of a stress-strain curve corresponding to values between two strain points of 0.05% strain and 0.25% strain is defined as initial elastic modulus in tension, and an elastic modulus calculated from a slope of a stress-strain curve corresponding to values between two strain points of 0.25% strain and 10% strain is defined as intermediate elastic modulus in tension.

The chuck-to-chuck distance of the tensile tester was set to 1 cm, and a test piece was fixed to a fixing tool using 1 cm above and below. A tensile speed was set to 10 mm/min. The initial elastic modulus in tension of the suture of Example 1 ranged from 520 to 645 MPa, and was 589 MPa as an average for five points of the sample, and the intermediate elastic modulus in tension ranged from 175 to 296 MPa, and was 245 MPa as an average for five points of the sample. The initial elastic modulus in tension of the suture of Example 2 ranged from 328 to 599 MPa and was 492 MPa as an average for five points of the sample, and the intermediate elastic modulus in tension ranged from 105 to 166 MPa and was 144 MPa as an average for five points of the sample. The initial elastic modulus in tension of the suture of Example 3 ranged from 222 to 467 MPa and was 373 MPa on average, and the intermediate elastic modulus in tension ranged from 99 to 134 MPa and was 116 MPa on average for five points of the sample. The initial elastic modulus in tension of the suture of Example 4 ranged from 354 to 484 MPa and was 391 MPa as an average for five points of the sample, and the intermediate elastic modulus in tension ranged from 139 to 184 MPa and was 167 MPa as an average for five points of the sample. The elastic modulus of the MonoMax suture of Comparative Example 1 has been reported to be 485 MPa (literature value, International Journal of Polymer Science, Vol.2012, Article ID216137), and even the actually measured initial elastic modulus in tension ranged from 576 to 626 MPa, and was 600 MPa as an average for three sample points, and the intermediate elastic modulus in tension ranged from 457 to 578 MPa, and was 531 MPa as an average for three sample points. The elastic moduli of PDSII sutures in Comparative Examples 2 and 3 have been reported to be 1370 MPa (literature values, International Journal of Polymer Science, Vol.2012, Article ID216137), and even the actually measured initial elastic modulus in tension of the suture of Comparative Example 2 ranged from 1480 to 1660 MPa, and was 1560 MPa as an average for three sample points, and the intermediate elastic modulus in tension ranged from 1140 to 1210 MPa, and was 1180 MPa as an average for three sample points. With respect to the actually measured elastic moduli of the suture of Comparative Example 3, the initial elastic modulus in tension ranged from 1680 to 1710 MPa and was 1710 MPa as an average for three sample points, and the intermediate elastic modulus in tension ranged from 1050 to 1080 MPa and was 1070 MPa as an average for three sample points. The actually measured initial elastic modulus in tension of the nylon suture of Comparative Example 4 (nylon suture 4-0 available from Nesco Suture was used for elastic modulus evaluation) ranged from 1250 to 1450 MPa, and was 1350 MPa as an average for three sample points, and the intermediate elastic modulus in tension ranged from 1020 to 1090 MPa and was 1040 MPa as an average for three sample points. Vicryl described in Comparative Example 5 is a braided yarn. When the initial elastic modulus in tension of 3-0 yarns was calculated on the assumption that the yarn is a monofilament yarn having a circular cross section, an average was 10000 MPa (actually measurement value) for three sample points, and the intermediate elastic modulus in tension was 4460 MPa as an average for three sample points. The elastic moduli are summarized in Table 10.

As described above, when the initial elastic moduli in tension of the P(3HB-co-4HB) stretchable sutures of Examples 1 to 4 are compared, the initial elastic modulus in tension is 589 MPa in Example 1, 492 MPa in Example 2, 373 MPa in Example 3, and 391 MPa in Example 4, but ranges from 1370 MPa to 1710 MPa for the PDSII sutures of Comparative Examples 2 and 3, is 1350 MPa for the nylon of Comparative Example 4, and 10000 MPa for Vicryl of Comparative Example 5 on the assumption that it is a braided but monofilament yarn. From a comparison among these, it can be said that the initial elastic moduli in tension of P(3HB-co-4HB) in Examples 1 to 4 are sufficiently lower than those in Comparative Examples 2 to 5. However, the initial elastic modulus in tension of the MonoMax suture ranges from about 485 MPa to 600 MPa, and the initial elastic moduli in tension of the MonoMax sutures in Examples 1 and 2 are almost equivalent to that of the MonoMax suture in Comparative Example 1. On the other hand, the intermediate elastic modulus in tension of Example 1 is 245 MPa and the intermediate elastic modulus in tension of Example 2 is 144 MPa, whereas the intermediate elastic modulus in tension of the MonoMax suture is 531 MPa. The intermediate elastic modulus in tension of P(3HB-co-4HB) is considerably lower than that of the MonoMax suture, and it can be seem that, when the strain increases from 0.25% to 10%, the P(3HB-co-4HB) stretchable sutures further exhibit the property of being easily elongated. At the same time, the P(3HB-co-4HB) stretchable suture has a property of returning to its original state even when it is elongated, and thus, when a knot is formed, it is appropriately elongated and the knot is formed by a thin portion of the elongated suture. The suture portions other than the knot are shrunk and tend to return to its original thickness. Therefore, hard-to-unravel ligation providing a small knot is considered to be possible. The intermediate tensile elastic moduli of the sutures of Examples 3 and 4 are 116 MPa and 167 Pa, which are also low as in Examples 1 and 2.

By using the starting polymer used in Example 4 and changing the spinning conditions (screw temperature, spinneret temperature, discharge amount, crystallization temperature, crystallization time, and draw ratio), heat treatment temperature (annealing step), and the like, a yarn having an initial elastic modulus in tension of from approximately 180 MPa to 500 MPa could be produced. By further changing the molecular weight and composition of the polymer to be used, the possibility of spinning which allows coverage of a wider range of elastic moduli is presumed.

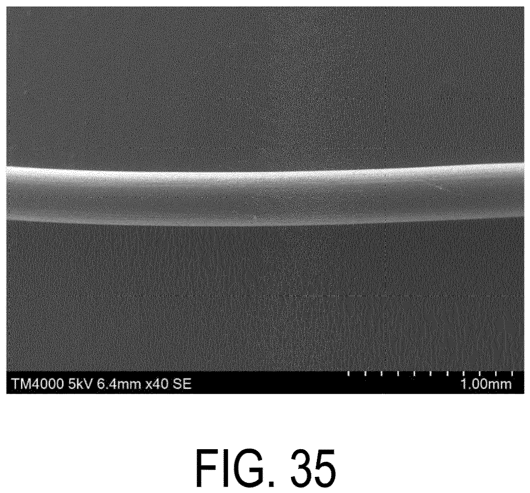

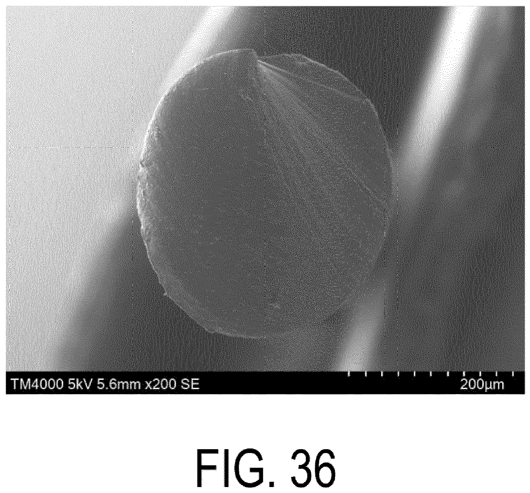

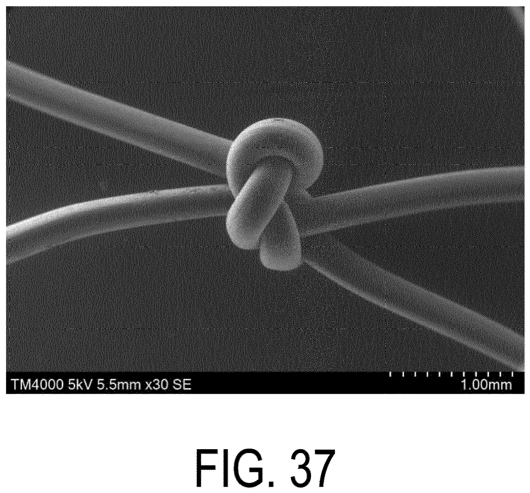

FIGS. 35 and 36 show examples of the fiber provided by industrial spinning and also shown in Example 4. FIG. 37 shows a state where a surgical knot is tied. It was observed that there was no hole inside the fiber, but that the yarn had an initial elastic modulus in tension of 391 MPa and an intermediate elastic modulus in tension of 167 Pa, and was made of a soft fiber and tolerant to elongation, and had a shrinking property, and that the knot was tight without capillaries.

As described above, the stretchable bioabsorbable fibrous medical material of the present invention allows easy knot formation, provides a small knot, and can reduce the number of knots. Therefore, the burden on a doctor during surgery is reduced, and, for a patient, physical irritation to tissue is reduced, which is useful for contribution to medical care.

<Summary of Examples and Comparative Examples>

The above-described examples and comparative examples are summarized in Table 10 below.

[Table 10-1]

| | Material | Elongation at break | Knot size *1 | KSF | Porosity | Initial elastic modulus*2 Strain 0.05% to 0.25% | Intermediate elastic modulus Strain 0.25% to 10% | Residual strain rate after 100% strain | Diameter of largest fine pore | Major axis length/minor axis length Cross-sectional shape |
|---|---|---|---|---|---|---|---|---|---|---|
| Example 1 3-0 size | P(3HB-co-14.8 mol% 4HB), Mw 320,000, | 240% 180% < (180 to 282%) | 2.45 | 1 | 25 to 55% | 589 MPa 520 to 645 | 245 MPa 175 to 296 | 40% | 100 μm | 1.7 (1.2 to 2.1) Ellipse |
| Example 2 3-0 size | P(3HB-co-15.3 mol% 4HB), Mw 470,000, | 183% 157% < (157 to 209%) | 3.59 | 2 | 0% | 492 MPa 328 to 599 | 144 MPa 105 to 166 | 33% | None | < 1.2 Circular |
| Example 3 1 size | P(3HB-co-15.3 mol% 4HB), Mw 450,000 | 250% 178% < 178 to 338 | 3.33 | 2 | 0% | 373 MPa 222 to 467 | 116 MPa 99 to 134 | 30% | None | < 1.2 Circular |

[Table 10-2]

| | Material | Elongation at break | Knot size *1 | KSF | Porosity | Initial elastic modulus*2 Strain 0.05% to 0.25% | Intermediate elastic modulus Strain 0.25% to 10% | Residual strain rate after 100% strain | Diameter of largest fine pore | Major axis length/ minor axis length Cross-sectional shape |
|---|---|---|---|---|---|---|---|---|---|---|
| Example 4 2.5-0 size | P(3HB-co-16.0 mol% 4HB), Mw 350,000 | 232% 192% < (192 to 272%) | 3.28 | 2 | 0% | 391 MPa 354 to 484 | 167 MPa 139 to 184 | 26% | None | < 1.2 Circular |
| Comparative Example 1 2-0 size | P(4HB) | 35 to 90 | 7.50 | 2 or 3 | 0% | (485 MPa) 600 MPa 576 to 626 | 531 MPa 457 to 578 | Break | None | < 1.2 Circular |
| Comparative Example 2 3-0 size | Polydioxanone | 45 to 50 | 7.82 | 3 | 0% | (1370 MPa) 1560 MPa 1480 to 1660 | 1180 MPa 1140 to 1210 | | None | < 1.2 Circular |
| Comparative Example 3 4-0 size | Polydioxanone | 45 to 50 | 7.33 | 3 | 0% | (1370 MPa) 1710 MPa 1680 to 1710 | 1070 MPa 1050 to 1080 | Break | None | < 1.2 Circular |
| Comparative Example 4 3-0 or 4-0 size | Nylon | 46 | - | 3 | 0% | 1350 MPa 1250 to 1450 | 1040 MPa 1020 to 1090 | Break | None | < 1.2 Circular |
| Comparative Example 5 3-0 or 4-0 size | Polyglycolic acid/poly L-lactic acid | 21 to 24 | - | 4 or 3 | 0% | 10000 MPa | 4460 MPa | Break | None | < 1.2 Circular |

*1 Knot size: average value of [area surrounded by perimeter/yarn diameter]
*2 With respect to the elastic modulus, the actually measured values are shown without parentheses and the literature values are shown with parentheses.

Examples 1 to 4 and Comparative Examples 1 to 4 are monofilaments, and Comparative Example 5 is a braid.

**Claims**

1. A fibrous medical material which is a fibrous material comprising a molded article provided by spinning and drawing a bioabsorbable aliphatic polymer, wherein an intermediate elastic modulus in tension at a strain ranging from 0.25% to 10% is lower than an initial elastic modulus in tension at a strain ranging from 0.05% to 0.25%, the intermediate elastic modulus in tension is 400 MPa or less, and a residual strain rate after 100% deformation is 70% or less, wherein

   the bioabsorbable aliphatic polymer is a polyhydroxyalkanoate, wherein
   the polyhydroxyalkanoate is a polyhydroxyalkanoate composed of two or more types of hydroxyalkanoate units, wherein
   the hydroxyalkanoate units contain 3-hydroxybutyrate and 4-hydroxybuyrate, wherein
   the proportion of the 4-hydroxybutyrate unit relative to all monomer units is 10 mol% to 30 mol%, wherein
   a weight average molecular weight of the polyhydroxyalkanoate after spinning is 300,000 or greater,

   wherein

   the elongation at break of the fibrous medical material is 180% or greater, wherein spinning of the bioabsorbable aliphatic polymer is performed by melt spinning, wherein drawing of the bioabsorbable aliphatic polymer is applied in a state in which microcrystals are present, and wherein the elongation at break, the elastic modulus, the residual strain, and the weight average molecular weight are measured according to the measurement methods defined in the description.

2. The fibrous medical material according to claim 1, wherein the initial elastic modulus in tension is 1000 MPa or less, wherein
   the elastic modulus is measured according to the measurement method defined in the description.

3. The fibrous medical material according to claim 1 or 2, wherein the initial elastic modulus in tension is 480 MPa or less, wherein
   the elastic modulus is measured according to the measurement method defined in the description.

4. The fibrous medical material according to any one of claims 1 to 3, wherein the intermediate elastic modulus in tension is 300 MPa or less, wherein
   the elastic modulus is measured according to the measurement method defined in the description.

5. The fibrous medical material according to any one of claims 1 to 4, wherein the residual strain rate after 100% deformation is 50% or less, wherein
   the residual strain is measured according to the measurement method defined in the description.

6. The fibrous medical material according to any one of claims 1 to 5, wherein a porosity is from 0% to 55%, wherein the porosity is measured according to the measurement method defined in the description.

7. The fibrous medical material according to any one of claims 1 to 6, wherein a diameter of a largest fine pore, as measured by microscopic observation of a cross section orthogonal to a fiber axis direction, is 100 $\mu$m or less, wherein the diameter of the largest fine pore is further measured according to the measurement method defined in the description.

8. The fibrous medical material according to any one of claims 1 to 7, wherein a ratio of a major axis length to a minor axis length (major axis length/minor axis length) in a cross section in a width direction is 1.0 or greater and 3.0 or less, wherein the minor axis length and the major axis length are measured according to the measurement method defined in the description.

**Patentansprüche**

1. Faseriges medizinisches Material, das ein faseriges Material ist, umfassend einen Formgegenstand, der durch Spinnen und Ziehen eines bioabsorbierbaren aliphatischen Polymers bereitgestellt wird, wobei

ein mittlerer Elastizitätsmodul bei Zugspannung bei einer Dehnung im Bereich von 0,25 % bis 10 % niedriger ist als ein anfänglicher Elastizitätsmodul bei Zugspannung bei einer Dehnung im Bereich von 0,05 % bis 0,25 %, der mittlere Elastizitätsmodul bei Zugspannung 400 MPa oder weniger beträgt und eine verbleibende Dehnungsrate nach 100 % Deformation 70 % oder weniger beträgt,

wobei das bioabsorbierbare aliphatische Polymer ein Polyhydroxyalkanoat ist,

wobei das Polyhydroxyalkanoat ein Polyhydroxyalkanoat ist, das aus zwei oder mehr Arten von Hydroxyalkanoateinheiten aufgebaut ist,

wobei die Hydroxyalkanoat-Einheiten 3-Hydroxybutyrat und 4-Hydroxybutyrat enthalten, wobei der Anteil der 4-Hydroxybutyrat-Einheit relativ zu sämtlichen Monomereinheiten 10 Mol-% bis 30 Mol-% beträgt,

wobei das gewichtsmittlere Molekulargewicht des Polyhydroxyalkanoats nach dem Spinnen 300.000 oder mehr beträgt,

wobei die Bruchdehnung des faserigen medizinischen Materials 180 % oder mehr beträgt, wobei das Spinnen des bioabsorbierbaren aliphatischen Polymers durch Schmelzspinnen durchgeführt worden ist,

wobei das Ziehen des bioabsorbierbaren aliphatischen Polymers in einem Zustand angewendet wird, in dem Mikrokristalle vorhanden sind, und

wobei die Bruchdehnung, der Elastizitätsmodul, die verbleibende Dehnung und das gewichtsmittlere Molekulargewicht gemäß den in der Beschreibung definierten Messmethoden gemessen werden.

2. Faseriges medizinisches Material gemäß Anspruch 1, wobei der anfängliche Elastizitätsmodul bei Zugspannung 1000 MPa oder weniger beträgt, wobei der Elastizitätsmodul gemäß der in der Beschreibung definierten Messmethode gemessen wird.

3. Faseriges medizinisches Material nach Anspruch 1 oder 2, wobei der anfängliche Elastizitätsmodul bei Zugspannung 480 MPa oder weniger beträgt, wobei der Elastizitätsmodul gemäß der in der Beschreibung definierten Messmethode gemessen wird.

4. Faseriges medizinisches Material gemäß einem der Ansprüche 1 bis 3, wobei der mittlere Elastizitätsmodul bei Zugspannung 300 MPa oder weniger beträgt, wobei der Elastizitätsmodul gemäß der in der Beschreibung definierten Messmethode gemessen wird.

5. Faseriges medizinisches Material nach einem der Ansprüche 1 bis 4, wobei die verbleibende Dehnungsrate nach 100%iger Deformation 50% oder weniger beträgt, wobei die verbleibende Dehnung gemäß der in der Beschreibung definierten Messmethode gemessen wird.

6. Faseriges medizinisches Material gemäß einem der Ansprüche 1 bis 5, wobei die Porosität 0 % bis 55 % beträgt, wobei die Porosität gemäß der in der Beschreibung definierten Messmethode gemessen wird.

7. Faseriges medizinisches Material gemäß einem der Ansprüche 1 bis 6, wobei der Durchmesser einer größten feinen Pore, gemessen durch mikroskopische Beobachtung eines Querschnitts orthogonal zur Richtung der Faserachse, 100 $\mu$m oder weniger beträgt, wobei der Durchmesser der größten feinen Pore gemäß der in der Beschreibung definierten Messmethode gemessen wird.

8. Faseriges medizinisches Material gemäß einem der Ansprüche 1 bis 7, wobei ein Verhältnis einer Hauptachsenlänge zu einer Nebenachsenlänge (Hauptachsenlänge/Nebenachsenlänge) in einem Querschnitt in einer Breitenrichtung 1,0 oder mehr und 3,0 oder weniger beträgt, wobei die Nebenachsenlänge und die Hauptachsenlänge gemäß dem in der Beschreibung definierten Messverfahren gemessen werden.

**Revendications**

1. Matière médicale fibreuse, laquelle est une matière fibreuse comprenant un article moulé obtenu par filage et étirage d'un polymère aliphatique bioabsorbable,

dans laquelle un module d'élasticité intermédiaire en tension, à une contrainte comprise entre 0,25 % et 10 %, est inférieur à un module d'élasticité initial en tension à une contrainte comprise entre 0,05 % et 0,25 %, le module d'élasticité intermédiaire en tension est égal ou inférieur à 400 MPa, et un taux de contrainte résiduelle après une déformation de 100 % est égal ou inférieur à 70 %, dans laquelle

le polymère aliphatique bioabsorbable est un polyhydroxyalcanoate,

le polyhydroxyalcanoate est un polyhydroxyalcanoate constitué d'au moins deux types d'unités hydroxyalcanoate,

les unités hydroxyalcanoate contiennent du 3-hydroxybutyrate et du 4-hydroxybutyrate,

la proportion de l'unité 4-hydroxybutyrate par rapport à toutes les unités monomère est comprise entre 10 % en mole et 30 % en mole,

une masse moléculaire moyenne en poids du polyhydroxyalcanoate après le filage est égale ou supérieure à 300 000 ;

l'allongement à la rupture de la matière médicale fibreuse est égal ou supérieur à 180 %,

le filage du polymère aliphatique bioabsorbable est effectué au moyen d'un filage par fusion,

l'étirage du polymère aliphatique bioabsorbable est mis en œuvre dans un état dans lequel des microcristaux sont présents, et

l'allongement à la rupture, le module d'élasticité, la contrainte résiduelle et la masse moléculaire moyenne en poids sont tous mesurés selon les procédés de mesure définis dans la description.

2. La matière médicale fibreuse selon la revendication 1, dans laquelle le module d'élasticité initial en tension est égal ou inférieur à 1000 MPa, et

le module d'élasticité est mesuré selon le procédé de mesure défini dans la description.

3. La matière médicale fibreuse selon les revendications 1 ou 2, dans laquelle le module d'élasticité initial en tension est égal ou inférieur à 480 MPa, et

le module d'élasticité est mesuré selon le procédé de mesure défini dans la description.

4. La matière médicale fibreuse selon l'une quelconque des revendications 1 à 3, dans laquelle le module d'élasticité intermédiaire en tension est égal ou inférieur à 300 MPa, et

le module d'élasticité est mesuré selon le procédé de mesure défini dans la description.

5. La matière médicale fibreuse selon l'une quelconque des revendications 1 à 4, dans laquelle le taux de contrainte résiduelle après une déformation de 100 % est égal ou inférieur à 50 %, et

la contrainte résiduelle est mesurée selon le procédé de mesure défini dans la description.

6. La matière médicale fibreuse selon l'une quelconque des revendications 1 à 5, dans laquelle une porosité est comprise entre 0 % et 55 %, et dans laquelle la porosité est mesurée selon le procédé de mesure défini dans la description.

7. La matière médicale fibreuse selon l'une quelconque des revendications 1 à 6, dans laquelle un diamètre d'un pore fin le plus grand, tel que mesuré par observation microscopique d'une coupe transversale orthogonale à un sens d'un axe de la fibre, est égal ou inférieur à 100 μm, et dans laquelle le diamètre du pore fin le plus grand est mesuré en outre selon le procédé de mesure défini dans la description.

8. La matière médicale fibreuse selon l'une quelconque des revendications 1 à 7, dans laquelle un rapport d'une longueur d'un grand axe à une longueur d'un petit axe (longueur du grand axe/longueur du petit axe), dans une coupe transversale dans le sens de la largeur, est égal ou supérieur à 1,0 et égal ou inférieur à 3,0, et dans laquelle la longueur du petit axe et la longueur du grand axe sont mesurées selon le procédé de mesure défini dans la description.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

# FIG. 15

FIG. 16

# FIG. 17

FIG. 18

FIG. 19

0.2mm

# FIG. 20

FIG. 21

# FIG. 22

# FIG. 23

FIG. 24

# FIG. 25

FIG. 26

FIG. 27

FIG. 28

FIG. 29

FIG. 30

FIG. 31

FIG. 32

FIG. 33

TM4000 5kV 5.8mm x250 SE                    200µm

# FIG. 34

TM4000 5kV 6.4mm x40 SE                    1.00mm

# FIG. 35

TM4000 5kV 5.6mm x200 SE                    200µm

# FIG. 36

TM4000 5kV 5.5mm x30 SE                    1.00mm

# FIG. 37

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001149462 A
- JP 2011006496 A
- JP 4071661 B
- JP 2019505338 T
- JP 6336523 A
- US 6867247 B
- JP 5031144 B
- JP 2007525601 T
- JP 2003513130 T
- JP 2003513131 T
- JP 1048821 A
- WO 2004101002 A
- CN 102586936 A
- WO 2019044837 A
- JP 4061638 A
- JP 7177894 A
- WO 2004029266 A
- US 6245537 B
- JP 2020096145 A
- JP 2019090739 A

**Non-patent literature cited in the description**

- *BMC Surgery*, 2008, vol. 8
- *Journal of Polymer Science*, vol. 2012
- **ABE et al.** *Macromolecules*, 1995, vol. 28, 7630
- **HORI et al.** *Polymer*, 1995, vol. 36, 4703
- **GILDING et al.** *Polymer*, 1979, vol. 20, 1459
- **SAITO et al.** *Polymer International*, 1996, vol. 39, 169
- **ODERMATT et al.** *International Journal of Polymer Science*, vol. 2012
- *International Journal of Polymer Science*, vol. 2012
- **SILVER E et al.** *J.Oral.Maxillofac.Surg.*, July 2016, vol. 74 (7), 1304-1312
- **FUNAI et al.** Creation of physical property value database of biological tissue for biomechanical simulation and application thereof. *Reports of the Industrial Research Institute of Shizuoka Prefecture*, 2007, vol. 52, 33-37
- **WILLIAMS et al.** *Biomed Tech (Berl).*, October 2013, vol. 58 (5), 439-452
- **ALBERTSMEIER et al.** *Langenbecks Arch Surg*, 2012, vol. 397, 363-371